(19) Europäisches Patentamt
European Patent Office
Office européen des brevets

(11) **EP 3 214 216 B1**

(12) **EUROPEAN PATENT SPECIFICATION**

(45) Date of publication and mention of the grant of the patent:
**24.02.2021 Bulletin 2021/08**

(51) Int Cl.:
***D04H 3/16*** *(2006.01)*   ***D04H 1/559*** *(2012.01)*
***D04H 3/007*** *(2012.01)*

(21) Application number: **15855299.2**

(22) Date of filing: **30.10.2015**

(86) International application number:
**PCT/JP2015/080791**

(87) International publication number:
**WO 2016/068312 (06.05.2016 Gazette 2016/18)**

(54) **USE OF A SPUNBOND NON-WOVEN FABRIC AS A DRAPE OR MEDICAL CLOTHING, NON-WOVEN FABRIC LAMINATE, MEDICAL CLOTHING, AND DRAPE**

VERWENDUNG EINES SPINNVLIESSTOFFES ALS ABDECKTUCH UND MEDIZINISCHE KLEIDUNG, VLIESSTOFFLAMINAT, MEDIZINISCHE KLEIDUNG, UND ABDECKTUCH

UTILISATION D'UN NON-TISSÉ FILÉ-LIÉ COMME VÊTEMENT MÉDICAL ET DRAPÉ, STRATIFIÉ DE NON-TISSÉ, VÊTEMENT MÉDICAL, ET DRAPÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2014 JP 2014222017**

(43) Date of publication of application:
**06.09.2017 Bulletin 2017/36**

(73) Proprietor: **Mitsui Chemicals, Inc.**
**Minato-ku**
**Tokyo 105-7122 (JP)**

(72) Inventors:
• **ICHIKAWA, Taro**
  **Sodegaura-shi**
  **Chiba 299-0265 (JP)**
• **SEKIOKA, Yusuke**
  **Sodegaura-shi**
  **Chiba 299-0265 (JP)**
• **KAWABE, Kuniaki**
  **Sodegaura-shi**
  **Chiba 299-0265 (JP)**

(74) Representative: **Hoffmann Eitle**
**Patent- und Rechtsanwälte PartmbB**
**Arabellastraße 30**
**81925 München (DE)**

(56) References cited:
EP-A1- 1 039 007   EP-A1- 2 463 428
EP-A1- 2 644 763   WO-A1-00/22219
WO-A1-2012/070518   JP-A- H1 088 459
JP-A- H1 088 459   JP-A- H07 189 024
JP-A- 2004 209 470   JP-A- 2008 517 171
US-A1- 2008 038 982

**Description**

Technical Field

The present invention relates to the use of a spunbond nonwoven fabric as a drape or medical clothing, a nonwoven fabric layered body, a medical clothing, and a drape.

Background Art

Nonwoven fabrics including propylene-based polymers are superior to nonwoven fabrics including ethylene-based polymers in that they have favorable spinnability, which facilitates thin fiber formation, and also have well-balanced strength, flexibility, and barrier performance. As nonwoven fabrics including propylene-based polymers have the above properties, they are widely used as medical materials, sanitary materials, absorbable items, packaging materials for various items, support materials, and backing materials.

In particular, long fiber nonwoven fabrics including propylene-based polymers obtained by a spunbond method or a melt blowing method have excellent comprehensive performance including lightweight property, uniformity, strength, flexibility, and barrier performance, and therefore, they are used for medical materials (e.g., surgical gowns, drapes, masks, sheets, and gauze).

Here, medical materials require sterilization treatment before use for the purpose of infection prevention. Examples of a method of sterilization treatment include electron beam sterilization, gamma ray sterilization, gas sterilization, and steam sterilization. A radioactive cobalt source is used for gamma ray sterilization, and an ethylene oxide gas is mainly used for gas sterilization, which are problematic in handling. In addition, steam sterilization is problematic in certainty of sterilization. Therefore, in general, electron beam sterilization is considered to be an ideal method of safe and secure sterilization for a short period of time.

However, when polypropylene nonwoven fabrics are treated by electron beam sterilization or gamma ray sterilization, strength tends to decrease in proportion to irradiation strength, and at the same time, barrier performance against blood or the like tends to decrease. Accordingly, a method of gas sterilization is employed in practice.

There have been attempts to impart resistance to radiation with the addition of additives to propylene-based polymers as means of improving the above problems. For example, Document 1 (Japanese Patent No. 2633936) suggests a nonwoven fabric including a propylene-based polymer, which contains a long-chain fatty acid ester of 3,5-di-t-butyl-4-hydroxybenzoid acid.

In addition, Document 2 (Japanese Patent Application Laid-Open (JP-A) No. H02-215848) suggests a propylene-based resin composition having resistance to radiation, which contains 95% by mass to 99.5% by mass of a propylene-based resin and 5% by mass to 0.5% by mass of an ethylene-$\alpha$-olefin copolymer having a specific density and a specific number of side chain groups. Further, Document 3 (Japanese National-Phase Publication (JP-A) No. 2001-508813) suggests a product containing a 50% by mass to 99% by mass of a polypropylene blended with 1% to 50% by mass of a specific polyethylene.

In addition to the above, Document 4 (Japanese National-Phase Publication (JP-A) No. 2010-509461) discloses a polymer mixture containing a polyethylene and a polypropylene containing a Ziegler-Natta catalyst.

EP 2 463 428 A1 relates to a mixed fiber spunbonded nonwoven fabric which comprises 90 to 10% by weight of a long fiber of thermoplastic resin (A) that has been hydrophilization-treated and 10 to 90% by weight of a long fiber of thermoplastic elastomer (B) and which has a strength ratio [the ratio of a strength at 20% of an elongation at a maximum strength (elongation at a maximum point) to the maximum strength] in at least one direction of not more than 40% and a bulk density of 0. 10 to 0. 40 g/cm3.

US 2008/038982 A1 relates to a nonwoven fabric laminate including at least one meltblown nonwoven fabric layer and mixed-fiber spunbonded nonwoven fabric layers on both surfaces of the at least one meltblown nonwoven fabric layer, the mixed-fiber spunbonded nonwoven fabric layers each comprising mixed fibers including 10 to 90 wt % of continuous fibers of a thermoplastic elastomer (A) and 90 to 10 wt % of continuous fibers of a thermoplastic resin (B) other than the thermoplastic elastomer (A) ((A)+(B)=100 wt %).

JP H10-088459 A relates to a soft nonwoven fabric that can be prepared by mixing a low-melting or a low-softening olefin-based copolymer such as a copolymer composed of 85-99 wt.% of propylene and 1-1 5wt.% of ethylene or a terpolymer composed of 84-97 wt.% of propylene, 1-15 wt.% of 1-butene and 1-10 wt.% of ethylene as a first component with 2-20 wt.% calculated as concentration in yarn of a hydrocarbon-based lubricant such as a paraffin having 50-155°C melting point or softening point and 500-5,000 ppm of inorganic substance powder such as titanium oxide having 0.04-2$\mu$m average particle diameters. The copolymer and a crystalline thermoplastic resin such as a polypropylene or a polyethylene terephthalate are combined into an eccentric sheath-core type state or a side-by-side state to form the objective nonwoven fabric of filament by a spun bond method.

EP 1 039 007 A1 relates to a polyethylene nonwoven fabric produced by the meltblowing process using a resin composition comprising a polyethylene (A) and a polyethylene wax (B).

Document 1: Japanese Patent No. 2633936
Document 2: Japanese Patent Application Laid-Open (JP-A) No. H02-215848
Document 3: Japanese National-Phase Publication (JP-A) No. 2001-508813
Document 4: JP-ANo. 2010-509461
Document 5: International Publication WO2014/046070

SUMMARY OF INVENTION

PROBLEMS TO BE SOLVED BY THE INVENTION

However, the above nonwoven fabric obtained by the technique disclosed in Japanese Patent No. 2633936 is prevented from having a decrease in strength after radiation irradiation, compared with a case in which a long-chain fatty acid ester compound of 3,5-di-t-butyl-4-hydroxybenzoic acid is not contained. Nevertheless, a decrease in strength after radiation irradiation is not sufficiently prevented to a satisfactory extent.

Further, a resin composition containing a propylene-based polymer mixed with an ethylene-based polymer is used for nonwoven fabrics obtained by the techniques disclosed in JP-ANo. H02-215848 and JP-ANo. 2001-508813 described above. The present inventors' findings revealed that in a case in which any of the resin compositions disclosed in the above documents is used, spinnability deteriorates. As a result, it is impossible to sufficiently thin fibers, which sometimes results in a case in which a favorable nonwoven fabric used as, for example, a medical material cannot be obtained. In addition, such nonwoven fabrics have a large decrease in strength after radiation irradiation, and the melting point of the above polyethylene is low. In particular, in a case in which the nonwoven fabrics are heat layered with films and the layered bodies are treated by secondary processing to obtain medical drape materials, they have not been suitable.

It would be possible to employ a method of using high-density polyethylene in order to improve heat resistance of the polyethylene in the above resin compositions including an ethylene-based polymer and a propylene-based polymer. As a nonwoven fabric obtained by such method, the above nonwoven fabric obtained by the technique disclosed in JP-ANo. 2010-509461 is specified as containing a specific high-density polyethylene for the improvement of strength. The inventors' findings, however, revealed that even in a case in which the above high-density polyethylene is added to a propylene-based polymer, the obtained mixture has poor spinnability. As a result, it is impossible to sufficiently thin fibers, which sometimes results in a case in which a favorable nonwoven fabric used as, for example, a medical material cannot be obtained.

In the case of the nonwoven fabric obtained by the technique disclosed in International Publication WO2014/046070 described above, a mixture of a high-density polyethylene and a propylene-based polymer is used so that spinnability of the mixture is improved. However, this nonwoven fabric contains fibers that are not sufficiently thinned and has high air permeability, and therefore, when it is layered as a medical material on a different member, it might result in lack of strength or barrier performance against blood or the like.

In view of the above, there has been no nonwoven fabric including fibers containing a propylene-based polymer, wherein strength is sufficiently high even after radiation sterilization treatment using an electron beam or the like, and even when the fiber diameter is reduced, aggravation of spinnability is prevented with the presence of an ethylene-based polymer.

An object of the invention is to provide a spunbond nonwoven fabric including fibers of a propylene-based polymer composition containing a propylene-based polymer, wherein sterilization treatment using an electron beam or the like is possible, strength is sufficiently high even after radiation sterilization treatment using an electron beam or the like, and even when the fiber diameter is reduced, aggravation of spinnability is prevented with the presence of an ethylene-based polymer, a nonwoven fabric layered body using the same, and medical clothing and drape using the same. Another object of the invention is to provide a melt blown nonwoven fabric including fibers of a propylene-based polymer composition containing a propylene-based polymer and a stabilizer, which is excellent when used as a member of a nonwoven fabric layered body in terms of inter-layer adhesion strength and strength of the nonwoven fabric layered body.

MEANS FOR SOLVING THE PROBLEMS

Means for solving the problems is described below.

<1> Use of a spunbond nonwoven fabric as a drape or medical clothing, wherein the spunbond nonwoven fabric consists of fibers of a propylene-based polymer composition containing 100 parts by mass of a propylene-based polymer (A) and from 1 to 10 parts by mass of an ethylene-based polymer (B) having a density of from 0.93 g/cm$^3$ to 0.98 g/cm$^3$,

wherein an air permeability of the spunbond nonwoven fabric, that is measured in accordance with JIS L 1096 (2010) by a Frazier type air permeability tester at a flow rate corresponding to a pressure differential of 125 Pa is 500 cm$^3$/cm$^2$/s or less.

<2> The use according to <1>, wherein the propylene-based polymer composition contains an ethylene-based polymer wax (C) having a weight-average molecular weight (Mw) of from 400 to 15,000 and a density of from 0.890 g/cm$^3$ to 0.980 g/cm$^3$.

<3> The use according to <2>, wherein a content of the ethylene-based polymer wax (C) is from 0.1 parts by mass to less than 4 parts by mass with respect to 100 parts by mass of the propylene-based polymer (A).

<4> The use according to any one of <1> to <3>, wherein a melt flow rate (MFR) of the ethylene-based polymer (B) is from 2 g/10 minutes to 25 g/10 minutes.

<5> The use according to <1>, wherein the spunbond nonwoven fabric has a weight per unit area of from 5 g/m$^2$ to 50 g/m$^2$.

<6> The use according to any one of <1> to <5>, wherein the mean fiber diameter of the fibers is 20 $\mu$m or less.

<7> The use according to any one of <1> to <6>, wherein a Strength INDEX of the spunbond nonwoven fabric after electron beam irradiation at an absorbed dose of 45 kGy is 10N or more.

<8> Use of a nonwoven fabric layered body as a drape or medical clothing, wherein the nonwoven fabric layered body includes the spunbond nonwoven fabric as defined in any one of <1> to <7>.

<9> The use according to <8>, wherein the nonwoven fabric layered body includes a melt blown nonwoven fabric including fibers of an ethylene-based polymer composition.

<10> The use according to <9>, wherein the ethylene-based polymer composition contains an ethylene-based polymer (a) having a melt flow rate (MFR) of from 10 g/10 minutes to 250 g/10 minutes and an ethylene-based polymer wax (b) having a weight-average molecular weight (Mw) of from 400 to 15000 at a (a):(b) mass ratio of from 90:10 to 10:90.

<11> The use according to <8>, wherein the nonwoven fabric layered body includes a melt blown nonwoven fabric including fibers of a propylene-based polymer composition containing a propylene-based polymer (c) and at least one stabilizer selected from the group consisting of an organophosphorous compound, a hindered amine-based compound, and a phenolic compound.

<12> A nonwoven fabric layered body comprising a spunbond nonwoven fabric as described in any one of <1> to <8> and a melt blown nonwoven fabric, wherein the melt blown nonwoven fabric comprises fibers of a propylene-based polymer composition containing a propylene-based polymer (c) and at least one stabilizer selected from the group consisting of an organophosphorous compound and a hindered amine-based compound.

<13> The nonwoven fabric layered body according to <12>, wherein the stabilizer contains an organophosphorous compound, a hindered amine-based compound, and a phenolic compound.

<14> An article of medical clothing or a drape, including the nonwoven fabric layered body according to <12> or <13>.

EFFECT OF THE INVENTION

According to the invention, a spunbond nonwoven fabric including fibers of a propylene-based polymer composition containing a propylene-based polymer, wherein sterilization treatment using an electron beam or the like is possible, strength is sufficiently high even after radiation sterilization treatment using an electron beam or the like, and even when the fiber diameter is reduced, aggravation of spinnability due to the presence of an ethylene-based polymer is prevented, a nonwoven fabric layered body using the same, and an article of medical clothing and drape using the same can be provided. Further, a melt blown nonwoven fabric including fibers of a propylene-based polymer composition containing a propylene-based polymer and a stabilizer, which is excellent when used as a member of a nonwoven fabric layered body in terms of inter-layer adhesion strength and strength of the nonwoven fabric layered body, can be provided.

DESCRIPTION OF EMBODIMENTS

Embodiments of the invention are described below.

Any numerical range expressed herein using the word "to" means a range of from a value corresponding to the minimum "to" a value corresponding to the maximum.

<Spunbond Nonwoven Fabric>

The spunbond nonwoven fabric of the invention consists of fibers of a propylene-based polymer composition containing 100 parts by mass of a propylene-based polymer (A) and from 1 part by mass to 10 parts by mass of an ethylene-based polymer (B) having a density of from 0.93 g/cm$^3$ to 0.98 g/cm$^3$. In addition, air permeability determined by a JIS L 1096 (2010) Frazier type air permeability tester under flow rate conditions at a pressure differential of 125 Pa is 500 cm$^3$/cm$^2$/s or less.

In another embodiment of the invention, the propylene-based polymer composition may further contain an ethylene-based polymer wax (C).

According to the invention, a spunbond nonwoven fabric including fibers of a propylene-based polymer composition containing a propylene-based polymer can be obtained, the spunbond nonwoven fabric having the above configuration, thereby realizing that sterilization treatment using an electron beam or the like is possible, strength is sufficiently high even after radiation sterilization treatment using an electron beam or the like, and even when the fiber diameter is reduced, aggravation of spinnability due to the presence of an ethylene-based polymer is prevented.

Further, such spunbond nonwoven fabric has a thin fiber diameter, and therefore, a nonwoven fabric layered body containing the spunbond nonwoven fabric can be preferably applied particularly to articles of medical clothing and drapes.

Hereafter, constituents of the invention are specifically described.

<Propylene-based Polymer (A)>

An propylene-based polymer (A) which is a major component of the propylene-based polymer composition used as a starting material of the spunbond nonwoven fabric of the invention is a polymer mainly containing propylene such as a propylene homopolymer or a copolymer of propylene and a small amount of an α-olefin. Examples of an α-olefin that is copolymerized with propylene include α-olefins with 2 carbons or more (other than 3 carbons) and preferably with from 2 to 8 carbons (other than 3 carbons). Specific examples thereof include α-olefins such as ethylene, 1-butene, 1-pentene, 1-hexene, 1-octene, and 4-methyl-1-pentene. One type of or two or more types of these α-olefins may be used. In order to maintain strength or spinning stability of a nonwoven fabric, the propylene-based polymer (A) is preferably a propylene homopolymer or a propylene·ethylene random copolymer. Further, in a case in which heat resistance is prioritized, the propylene-based polymer (A) is preferably a propylene homopolymer.

The ethylene content in the propylene·ethylene random copolymer is favorably from 0.5 mol% to 10 mol%, preferably from 3 mol% to 8 mol%, and more preferably from 4 mol% to 7 mol%.

The melting point (Tm) of the propylene-based polymer (A) is not particularly limited; however, it is favorably in a range of 125°C or more, preferably in a range of 140°C or more, still more preferably in a range of 155°C or more, and particularly preferably in a range of from 157°C to 165°C.

The melt flow rate (MFR$_{230}$: ASTM D-1238; 230°C; load: 2160 g) of the propylene-based polymer (A) according to the invention is not particularly limited as long as melt spinning of the propylene-based polymer (A) is possible; however, it is preferably in a range of from 1 g/10 minutes to 500 g/10 minutes, more preferably in a range of from 5 g/10 minutes to 200 g/10 minutes, and still more preferably in a range of from 10 g/10 minutes to 100 g/10 minutes.

In addition, a ratio (Mw/Mn) of the weight-average molecular weight (Mw) and the number average molecular weight (Mn) for the propylene-based polymer (A) according to the invention is preferably from 1.5 to 5.0. It is more preferably in a range of from 1.5 to 4.5 in that good spinnability is achieved and fibers having remarkably excellent fiber strength can be obtained. Mw and Mn can be determined by gel permeation chromatography (GPC) in accordance with a known method.

Further, the density of the propylene-based polymer (A) according to the invention is preferably in a range of from 0.895 g/cm$^3$ to 0.915 g/cm$^3$, more preferably in a range of from 0.900 g/cm$^3$ to 0.915 g/cm$^3$, and still more preferably in a range of from 0.905 g/cm$^3$ to 0.910 g/cm$^3$.

In the invention, density is determined to be a value obtained by measurement in accordance with the density gradient method of JIS K7112.

<Ethylene-based Polymer (B)>

An ethylene-based polymer (B) which is a component other than propylene-based polymer composition that is a starting material of the spunbond nonwoven fabric of the invention is an ethylene homopolymer or a copolymer of ethylene and a small amount of a different α-olefin. The ethylene-based polymer (B) is specifically a polymer mainly containing ethylene such as a high pressure low-density polyethylene, a linear low-density polyethylene (so-called LLDPE), a middle-density polyethylene (so-called MDPE), or a high-density polyethylene (so-called HDPE).

Examples of a different α-olefin that is copolymerized with ethylene include α-olefins with from 3 to 20 carbons such as propylene, 1-butene, 1-pentene, 1-hexene, 4-methyl-1-pentene, 1-octene, 1-decene, 1-dodecene, 1-tetradecene, 1-hexadecene, 1-octadecene, and 1-eicosene. The ethylene-based polymer (B) may be an ethylene homopolymer or a mixture containing two or more types.

An ethylene-based polymer having a density within a range of from 0.93 g/cm$^3$ to 0.98 g/cm$^3$ is used for the ethylene-based polymer (B) according to the invention. In a case in which an ethylene-based polymer having a density within the above range is used, aggravation of spinnability is prevented, resulting in excellent strength of the obtained spunbond nonwoven fabric. In addition, excellent temporal stability is also achieved. The range of from 0.940 $g/cm^3$ to 0.980$g/cm^3$ is preferable and the range of from 0.940 $g/cm^3$ to 0.975 $g/cm^3$ is still more preferable in that aggravation of spinnability is further prevented and the strength is further improved. High-density polyethylene (HDPE) having a density within a range of from 0.950 $g/cm^3$ to 0.970 $g/cm^3$ is particularly preferable.

The melt flow rate ($MFR_{190}$: ASTM D 1238; 190°C; load: 2160 g) of the ethylene-based polymer (B) according to the invention is not particularly limited as long as melt spinning of the ethylene-based polymer (B) mixed with the propylene-based polymer (A) is possible. In terms of the spinnability of fibers, fiber diameter, and strength, the melt flow rate of the ethylene-based polymer (B) is preferably in a range of from 1 g/10 minutes to 50 g/10 minutes, more preferably in a range of from 2 g/10 minutes to 25 g/10 minutes, and still more preferably in a range of from 2 g/10 minutes to 10 g/10 minutes.

In a case in which the melt flow rate of the ethylene-based polymer (B) is in the above range, spinnability of the propylene-based polymer composition is favorable, and the obtained spunbond nonwoven fabric has excellent strength. As these properties can be realized, the obtained spunbond nonwoven fabric can be favorably used particularly for medical materials and the like.

Polymers obtained by a variety of known manufacturing methods (e.g., the high-pressure method and the middle- or low-pressure method using a Ziegler catalyst or a metallocene catalyst) can be used for the ethylene-based polymer (B) according to the invention. Among these polymers, in a case in which an ethylene-based polymer obtained through polymerization using a metallocene-based catalyst is used, favorable spinnability of a propylene-based polymer composition is achieved, and favorable strength or the like of the obtained spunbond nonwoven fabric is achieved, which is preferable.

<Ethylene-based Polymer Wax (C)>

The propylene-based polymer composition that is a starting material of the spunbond nonwoven fabric of the invention may contain an ethylene-based polymer wax (C) in another embodiment of the invention, if necessary.

The ethylene-based polymer wax (C) has effects of improving dispersibility of the propylene-based polymer (A) and the ethylene-based polymer (B). This facilitates the further improvement of spinnability. Therefore, it is preferable for the propylene-based polymer composition to contain the ethylene-based polymer wax (C).

Note that the ethylene-based polymer wax (C) according to the invention has a molecular weight lower than that of the ethylene-based polymer (B), meaning that the ethylene-based polymer wax (C) is a wax-type polymer.

The weight-average molecular weight (Mw) of the ethylene-based polymer wax (C) is preferably in a range of less than 15,000, more preferably in a range of less than 15,000, still more preferably in a range of 10,000 or less, still more preferably in a range of 6,000 or less, still more preferably in a range of less than 6,000, particularly preferably in a range of 5,000 or less, and most preferably in a range of 1,500 or less. The lower limit thereof is preferably 400 or more and more preferably 1,000 or more. In a case in which the weight-average molecular weight (Mw) of the ethylene-based polymer wax (C) is within the above range, it is easier to improve spinnability of the propylene-based polymer composition, to reduce the fiber diameter, and to achieve temporal stability. In a case in which a nonwoven fabric layered body is formed by layering the spunbond nonwoven fabric of the invention with, for example, a melt blown nonwoven fabric including an ethylene-based polymer, it is easier to achieve excellent inter-layer adhesiveness and strength, which is also preferable.

The weight-average molecular weight (Mw) of the ethylene-based polymer wax (C) according to the invention is a value obtained by GPC measurement under the following conditions. The weight-average molecular weight is calculated in accordance with the conversion method described below using a calibration curve created with a commercially available standard monodisperse polystyrene.

Apparatus: Alliance GPC 2000 gel permeation chromatograph (manufactured by Waters) Solvent: o-dichlorobenzene
Column: TSKgel GMH6-HT × 2, TSKgel GMH6-HTL column × 2 (each manufactured by Tosoh Corporation)
Flow rate: 1.0 mL/minute
Sample: 0.15 mg/mL o-dichlorobenzene solution
Temperature: 140°C
Molecular weight conversion: PE conversion/universal calibration method

The following coefficients in the Mark-Houwink viscosity equation were used for calculation for universal calibration.

Coefficient of polystyrene (PS): KPS=1.38 $\times$ 10$^{-4}$, aPS=0.70
Coefficient of polyethylene (PE): KPE=5.06 $\times$ 10$^{-4}$, aPE=0.70

The softening point of the ethylene-based polymer wax (C) according to the invention, which is determined in accordance with JIS K2207, is preferably in a range of from 90°C to 145°C, more preferably in a range of from 90°C to 130°C, still more preferably in a range of from 100°C to 120°C, and most preferably in a range of from 105°C to 115°C.

The ethylene-based polymer wax (C) according to the invention may be either an ethylene homopolymer or a copolymer of ethylene and an $\alpha$-olefin with from 3 to 20 carbons. In a case in which a copolymer of ethylene and an $\alpha$-olefin with from 3 to 20 carbons is uses as the ethylene-based polymer wax (C), the number of carbons for the $\alpha$-olefin that is copolymerized with ethylene is more preferably from 3 to 8 carbons, still more preferably from 3 to 4 carbons, and particularly preferably 3 carbons. When the number of carbons for the $\alpha$-olefin that is copolymerized with ethylene is within the above range, favorable spinnability is achieved, thereby making it possible to improve nonwoven fabric properties such as strength. The reason for such improvement is unclear but it is probably as follows. In the ethylene-based polymer wax (C) according to the invention, when the number of carbons of the $\alpha$-olefin that is copolymerized with ethylene is within the above range, it would facilitate dispersion of the ethylene-based polymer (B) in the propylene-based polymer (A) via the ethylene-based polymer wax (C). That is, it is considered that as the ethylene-based polymer wax (C) functions as a compatibilizing agent for the propylene-based polymer (A) and the ethylene-based polymer (B), uniformity of the propylene-based polymer composition of the invention is enhanced, resulting in the improvement of the balance of properties such as spinnability and strength of a nonwoven fabric.

In a case in which an ethylene homopolymer is used as the ethylene-based polymer wax (C), it has an excellent property of being kneaded with the ethylene-based polymer (B) and excellent spinnability. The ethylene-based polymer wax may be used alone or a mixture of two or more types thereof may be used.

The ethylene-based polymer wax (C) according to the invention may be manufactured without particular limitation by any method such as a generally used manufacturing method of polymerization of a low-molecular-weight polymer or a method including reducing the molecular weight through heat degradation of a high-molecular-weight ethylene-based polymer.

In addition, the ethylene-based polymer wax (C) according to the invention may be purified by a method of solvent separation based on a difference in solubility in a solvent, distillation, or the like.

In a case in which the ethylene-based polymer wax (C) is obtained by a generally used manufacturing method of polymerization of a low-molecular-weight polymer, it can be manufactured by a variety of known manufacturing methods such as a manufacturing method of polymerization using a Ziegler-Natta catalyst or a metallocene-based catalyst described in JP-ANo. H08-239414 or International Publication WO2007/114102.

The density of the ethylene-based polymer wax (C) according to the invention is not particularly limited; however, it is preferably from 0.890 g/cm$^3$ to 0.980 g/cm$^3$, more preferably 0.910 g/cm$^3$ or more, and still more preferably 0.920 g/cm$^3$ or more. It is also preferably 0.960 g/cm$^3$ or less and more preferably 0.950 g/cm$^3$ or less. When the ethylene-based polymer wax (C) having a density within the above range is used, the propylene-based polymer composition tends to have excellent spinnability. In addition, in a case in which a nonwoven fabric layered body is formed by layering the spunbond nonwoven fabric of the invention with, for example, a melt blown nonwoven fabric including an ethylene-based polymer or the like, inter-layer adhesiveness or strength tends to be excellent, which is also preferable.

In the invention, the difference between the density of the propylene-based polymer (A) and the density of the ethylene-based polymer wax (C) is not particularly limited; however, it is more preferably less than 0.35 g/cm$^3$, particularly preferably less than 0.20 g/cm$^3$, and most preferably less than 0.15 g/cm$^3$. When the density difference is within the above range, favorable spinnability is achieved, thereby making it possible to improve nonwoven fabric properties such as strength. The reason for such improvement is unclear but it is probably as follows. When the difference between the density of the propylene-based polymer (A) and the density of the ethylene-based polymer wax (C) according to the invention is within the above range, it would facilitate dispersion of the ethylene-based polymer (B) in the propylene-based polymer (A) via the ethylene-based polymer wax (C). That is, it is considered that as the ethylene-based polymer wax (C) functions as a compatibilizing agent for the propylene-based polymer (A) and the ethylene-based polymer (B), uniformity of the propylene-based polymer composition of the invention is enhanced, resulting in the improvement of the balance of characteristics such as spinnability and strength of a nonwoven fabric.

<Propylene-based Polymer Composition>

As described above, the propylene-based polymer composition that is a starting material of the spunbond nonwoven fabric of the invention is a propylene-based polymer composition containing from 1 part by mass to 10 parts by mass of the ethylene-based polymer (B) with respect to 100 parts by mass of the propylene-based polymer (A).

In another embodiment of the invention, it may be a propylene-based polymer composition containing from 1 part by mass to 10 parts by mass the ethylene-based polymer (B) with respect to 100 parts by mass of the propylene-based polymer (A) and further containing the ethylene-based polymer wax (C).

The content of the ethylene-based polymer (B) is preferably 2 parts by mass or more and more preferably 3 parts by mass or more. It is also preferably 9 parts by mass or less and more preferably 8 parts by mass or less.

A spunbond nonwoven fabric obtained using the propylene-based polymer composition according to the embodiment has excellent strength after radiation sterilization using an electron beam or the like when the content of the ethylene-based polymer (B) is 1 part by mass or more. In addition, in a case in which such nonwoven fabric is used for a medical material in order to achieve sufficient durability [e.g., bonding strength or the like at a heat embossing site (also referred to as a thermal compression site)], excellent strength or flexibility can be achieved. Meanwhile, when the content of the ethylene-based polymer (B) is 10 parts by mass or less, the propylene-based polymer composition has excellent spinnability.

That is, in a case in which the content of the ethylene-based polymer (B) does not meet the above range, strength after radiation sterilization might decline or spinnability of the propylene-based polymer composition might deteriorate.

In addition, in a case in which the propylene-based polymer composition in the invention contains the ethylene-based polymer wax (C), dispersibility of the propylene-based polymer (A) and the ethylene-based polymer (B) is improved as described above, which eventually facilitates further improvement of spinnability. In consideration of enhancement of the effects of improving dispersion with the use of the ethylene-based polymer wax (C), thinning of fibers, and fiber strength, the content of the ethylene-based polymer wax (C) is preferably from 0.1 parts by mass to less than 4 parts by mass, more preferably from 0.5 parts by mass to 3 parts by mass, and still more preferably from 0.5 parts by mass to 2.5 parts by mass with respect to 100 parts by mass of the propylene-based polymer.

For manufacturing of the propylene-based polymer composition in the invention, the following examples of conventionally known catalysts can be favorably used: a magnesium-bearing titanium catalyst described in JP-ANo. S57-63310, JP-ANo. S58-83006, JP-ANo. H03-706, Japanese Patent No. 3476793, JP-ANo. H04-218508, or JP-ANo. 2003-105022; a metallocene catalyst described in International Publication WO2001/53369, International Publication WO2001/27124, JP-ANo. H03-193796, or JP-ANo. H02-41303.

The propylene-based polymer composition according to the invention may optionally contain other components other than the propylene-based polymer (A) and the ethylene-based polymer (B) within the range not impairing the objectives of the invention, if necessary. These components can be mixed using a variety of known methods.

Examples of other components that can be optionally used, if necessary, include a variety of the following additives: other polymers; colorants; antioxidants such as phosphorous or phenolic antioxidants; weathering stabilizers such as benzotriazole-based weathering stabilizers; light resistance stabilizers such as hindered amine-based light resistance stabilizers; antiblocking agents; dispersants such as calcium stearate; lubricants; nucleophiles; pigments; softeners; hydrophilic agents; water repellents; auxiliary agents; water repellents; fillers; antimicrobials; agricultural chemicals; insecticides; medicines; natural oil; and synthetic oil.

In particular, in a case in which stability in terms of strength, discoloration, odor, or the like is imparted through radiation irradiation using an electron beam or the like, it is effective to add an antioxidant, a weathering stabilizer, a light resistance stabilizer, a pigment, a refresher, or the like.

Examples of stabilizers that can be preferably used include: organophosphorous compounds such as tris(2,4-di-tert-butylphenyl)phosphite; hindered amine-based compounds such as decanedioic acid bis(2,2,6,6-tetramethyl-4-piperidinyl) and 2,4-dichloro-6-(1,1,3,3-tetramethylbutylamino)-1,3,5-triazine·N,N'-bis(2,2,6,6-tetramethyl-4-piperidyl)hexamethylenediamine polycondensate; and phenolic compounds such as 1,1,3-tris(2-methyl-4-hydroxy-5-t-butylphenyl)-butane. It is more preferable to use any combination of these stabilizers. Examples of more preferable stabilizers include: at least one hindered amine-based compound such as organophosphorous compounds of tris(2,4-di-tert-butylphenyl)phosphite, decanedioic acid bis(2,2,6,6-tetramethyl-4-piperidinyl), and 2,4-dichloro-6-(1,1,3,3-tetramethylbutylamino)-1,3,5-triazine·N,N'-bis(2,2,6,6-tetramethyl-4-piperidyl)hexamethylenediamine polycondensate; and phenolic compounds such as 1,1,3-tris(2-methyl-4-hydroxy-5-t-butylphenyl)-butane.

Examples thereof further include: metal salts of fatty acid such as zinc stearate, calcium stearate, and 1,2-hydroxycalcium stearate; and polyalcohol fatty acid esters such as glycerin monostearate, glycerin distearate, pentaerythritol monostearate, pentaerythritol distearate, and pentaerythritol tristearate. It is also possible to use any combination of these examples.

The following fillers may be added: silica, diatomaceous earth, alumina, titanium oxide, magnesium oxide, pumice powder, pumice balloon, aluminum hydroxide, magnesium hydroxide, basic magnesium carbonate, dolomite, calcium sulfate, potassium titanate, barium sulfate, calcium sulfite, talc, clay, mica, asbestos, calcium silicate, montmorillonite, bentonite, graphite, aluminum powder, and molybdenum sulfide.

According to an embodiment which is not part of the present invention, the spunbond nonwoven fabric may be a nonwoven fabric including a mixed fiber prepared by mixing a different type of fibers (e.g., fibers obtained from the propylene-based polymer (A) alone or fibers obtained from a different olefin-based polymer, polyester), in addition to fibers obtained from the propylene-based polymer composition described above, within the range not impairing the objectives of the invention. However, it does not comprise a fiber obtained from a thermoplastic elastomer.

Further, fibers forming the spunbond nonwoven fabric of the invention may be fibers including the above described propylene-based polymer composition alone, or may be composite fibers having a side-by-side or core-clad structure and including the propylene-based polymer composition within the range not impairing the objectives of the invention.

The cross-sectional shape of fibers forming the spunbond nonwoven fabric of the invention may be a circle shape, a polygonal shape such as a star, triangular, rectangular, or pentagonal shape, an ellipse shape, or a hollow shape.

<Physical Properties of Spunbond Nonwoven Fabric>

In a case in which an air permeability of the spunbond nonwoven fabric of the invention is determined by a JIS L 1096 (2010) Frazier type air permeability tester under flow rate conditions at a pressure differential of 125 Pa and a weight per unit area of 20 g/m2, the air permeability is 500 $cm^3/cm^2/s$ or less, preferably 450 $cm^3/cm^2/s$ or less, and more preferably 400 $cm^3/cm^2/s$ or less. In addition, the lower limit thereof is not particularly limited; however, it is 50 $cm^3/cm^2/s$ or more, preferably 100 $cm^3/cm^2/s$ or more, and more preferably 200 $cm^3/cm^2/s$ or more. When the air permeability of the spunbond nonwoven fabric is within the above range, appropriate air permeation or barrier performance can be achieved. In addition, the obtained spunbond nonwoven fabric has excellent strength. In a case in which the air permeability is within such range, as the spunbond nonwoven fabric of the invention has the above properties, it can be favorably used particularly for medical materials and the like.

The weight per unit area of the spunbond nonwoven fabric of the invention is preferably in a range of from 5 $g/m^2$ to 50 $g/m^2$ and more preferably in a range of from 10 $g/m^2$ to 25 $g/m^2$.

The mean fiber diameter of fibers forming the spunbond nonwoven fabric of the invention is preferably in a range of from 5 $\mu$m to 30 $\mu$m. The upper limit of the mean fiber diameter is more preferably 25 $\mu$m or less and still more preferably 20 $\mu$m or less. In particular, in a case in which the mean fiber diameter is 20 $\mu$m or less, fibers forming the nonwoven fabric are sufficiently thin, which can be favorably used particularly for medical materials and the like. In addition, the lower limit of the mean fiber diameter is not particularly limited; however, in a case in which the mean fiber diameter is 10 $\mu$m or more, a nonwoven fabric having excellent strength can be obtained.

Regarding strength of the spunbond nonwoven fabric of the invention in terms of strength after electron beam irradiation at an absorbed dose of 45 kGy at a weight per unit area of 20 $g/m^2$, MD (machine direction) strength is preferably 20N or more and CD (cross machine direction) strength is 10N or more. More preferably, MD strength is 25N or more and CD strength is 15N or more. When the strengthes are within the above ranges, the nonwoven fabric is unlikely to be perforated or broken, which is preferable.

Here, the absorbed dose means a radiation dose upon 1 joule energy absorption per kg, which is expressed by Gy.

In addition, when the weight per unit area is 20 $g/m^2$, Strength INDEX after electron beam irradiation at an absorbed dose of 45kGy is preferably 10N or more, still more preferably 13N or more, and most preferably 18N or more. When the spunbond nonwoven fabric of the invention has the Strength INDEX within the above range, it has sufficiently excellent strength even after electron beam irradiation. Further, when the spunbond nonwoven fabric of the invention has the Strength INDEX within the above range after electron beam irradiation, it has sufficiently excellent strength, and it can be favorably used particularly for medical materials and the like, which is preferable.

Here, Strength INDEX in the invention means a value calculated by the following equation.

$$[\text{Equation}]: \text{Strength INDEX} = ((((\text{MD strength})^2 + (\text{CD strength})^2))/2)^{0.5}$$

<Method of Manufacturing Spunbond Nonwoven Fabric>

The spunbond nonwoven fabric of the invention can be manufactured by a known method of manufacturing a spunbond nonwoven fabric. Specifically, it can be manufactured by, for example, conducting spinning of the above propylene-based polymer composition from a spinning nozzle in advance, cooling long fiber filaments that have been spun out using a refrigeration fluid or the like, applying tension to the filaments using air to stretch and result in a certain level of fineness, and collecting the obtained filaments on a running collection belt, thereby obtaining a spunbond nonwoven fabric including the filaments that have accumulated to a certain thickness.

In addition, the accumulated web can be treated by interlacing treatment, if necessary. Examples of an interlacing method that can be used, if appropriate, include a variety of methods such as a method of heat embossing treatment using a roll, a method of ultrasonic fusion; a method of interlacing fibers using water jet, a method of fusion in a hot air through system, and a method using a needle punch.

In addition, it is preferable to treat the spunbond nonwoven fabric of the invention by partial thermal compression via embossing or the like in order to improve strength of the obtained spunbond nonwoven fabric and maintain well-balanced flexibility and air permeation. In a case in which the spunbond nonwoven fabric of the invention is treated by thermal compression via heat embossing, the embossed area percentage (at a thermal compression site) is preferably in a range of from 5% to 30% and more preferably in a range of from 5% to 20%. Examples of the impressed shape include a circle shape, an ellipse shape, an oval shape, a square shape, a diamond shape, a rectangle shape, a quadrangle shape, a quilt shape, a lattice shape, a testudinal shape, and a continuous shape based on any of such shapes.

The spunbond nonwoven fabric of the invention may be treated by gear processing, printing, coating, laminating, heat treatment, or secondary processing such as shaping processing, hydrophilic processing, water-repellent processing, or press processing within the range not impairing the objectives of the invention. Secondary processing can be applicable to a nonwoven fabric layered body including the spunbond nonwoven fabric of the invention.

The spunbond nonwoven fabric of the invention may be treated by processing treatment such as water-repellent treatment, if necessary. Processing treatment such as water-repellent treatment can be carried out via coating with a processing agent such as a fluorinated water repellent or forming of a nonwoven fabric by mixing a water repellent as an additive with a resin starting material in advance. The amount of the attached water repellent (content) is adequately from 0.5% by mass to 5.0% by mass.

In addition, a method of imparting alcohol repellency is, for example, a method including attaching a fluorinated processing agent to the spunbond nonwoven fabric so that the amount of the attached fluorinated processing agent is from 0.01% by mass to 3% by mass. In this case, a method of attaching the processing agent and a drying method is not particularly limited. Examples of the method of adhering the processing agent include: a method including spraying the processing agent; a method including immersing the spunbond nonwoven fabric in a processing agent bath and squeezing the spunbond nonwoven fabric with a mangle; and a method including coating. Examples of the drying method include: a method using a hot air dryer; a method using a tenter; and a method including bringing the spunbond nonwoven fabric into contact with a heating element.

As a result of the above processing treatment, in a case in which the spunbond nonwoven fabric is used, for example, for a medical gown or the like, water, alcohol, or oil is unlikely to permeate through the spunbond nonwoven fabric. Thus, even in the case that alcohol disinfection is performed or attachment of blood or the like is occurred, high barrier performance is achieved. In addition, the above processing can be applied to a nonwoven fabric layered body containing the spunbond nonwoven fabric of the invention.

In addition, an antistatic property may be imparted to the spunbond nonwoven fabric of the invention. Examples of a method of imparting an antistatic property include a method of coating using an adequate agent of imparting an antistatic property (e.g., fatty acid ester or quaternary ammonium salt) and a method of forming a nonwoven fabric by mixing an adequate agent of imparting an antistatic property as an additive with a resin starting material. The antistatic property is preferably set to a level of 1,000V or less at 20°C in a 40%RH atmosphere in accordance with the method of JIS L1094C (provided that friction cloth is cotton cloth). The antistatic property is preferably set to a level of 1,000V or less at 20°C in an 40%RH atmosphere in accordance with the method of JIS L1094C (provided that friction cloth is cotton cloth).

By imparting the above antistatic property, it is possible to improve the wear comfort in a case in which, for example, the spunbond nonwoven fabric is used for a medical gown or the like. In addition, the above processing can be applied to a nonwoven fabric layered body including the spunbond nonwoven fabric of the invention.

The spunbond nonwoven fabric of the invention can be applied to, for example, various materials such as general sanitary materials, living materials, industrial materials, and medical materials. Specifically, it can be favorably used as a material for ground fabrics of disposable diapers, sanitary napkins, and compresses, bed covers, and the like. In addition, although the spunbond nonwoven fabric of the invention may be used alone, it is preferable to layer the spunbond nonwoven fabric with other materials described below in order to impart other functions. Further, the spunbond nonwoven fabric of the invention is stable against even a radioactive ray such as an electron beam irradiated upon sterilization or disinfection. Therefore, for example, a nonwoven fabric layered body of the spunbond nonwoven fabric of the invention and other materials can be favorably applied particularly to medical materials for gowns, caps, masks, isolation gown, patient clothes, drape, sheets, *kurumu* (sterile wrap), towels, and the like used in hospitals and the like, if necessary. Further, as the spunbond nonwoven fabric of the invention has favorable post-processing properties such as a heat-sealing property, it can be applied to general living materials for deoxidants, pocket warmers, warm compresses, masks, CD (compact disc) cases, food wrapping materials, clothes covers, and the like. For the same reason, the spunbond nonwoven fabric of the invention can be favorably used for vehicle interior materials and various backing materials. Since thin fibers constitute the spunbond nonwoven fabric of the invention, the spunbond nonwoven fabric of the invention can also be widely applied to filter materials for liquid filters, air filters, and the like.

<Nonwoven Fabric Layered Body>

The nonwoven fabric layered body in the first embodiment of the invention contains the above spunbond nonwoven fabric.

The nonwoven fabric layered body in the first embodiment of the invention can be formed by layering a material that is the same as or different from the spunbond nonwoven fabric of the invention, such as a nonwoven fabric or film, on at least one side of the spunbond nonwoven fabric for the purpose of, for example, imparting other functions (e.g., adjustment of the balance among uniformity, barrier performance, and air permeation, or weight saving).

Examples of a material that can be layered on the spunbond nonwoven fabric of the invention include a short fiber nonwoven fabric and a long fiber nonwoven fabric. Examples of fiber materials for these nonwoven fabrics include cellulosic fibers of cotton, cupra, and rayon; polyolefin-based fibers; polyamide-based fibers; and polyester-based fibers. In addition, examples of these nonwoven fabrics include the following materials: other spunbond nonwoven fabrics which are the same as or different from the spunbond nonwoven fabric of the invention; melt blown nonwoven fabrics; wet nonwoven fabrics; dry nonwoven fabrics; dry pulp nonwoven fabrics; flash-spun nonwoven fabrics; and spread nonwoven fabrics. Examples of the film include films of a polyolefin-based resin, a polyamide-based resin, and a polyester-based resin. One type of or two or more types of these materials can be selected and layered. For example, the spunbond nonwoven fabric of the invention, a melt blown nonwoven fabric, and the spunbond nonwoven fabric of the invention can be layered in that order, and a nonwoven fabric such as a dry nonwoven fabric can be further layered thereon.

In addition, the term "short fibers" used in the invention refers to fibers having a mean fiber length of about 200 mm or less. The term "long fibers" refers to "continuous long fibers (continuous filaments)" described in, for example, the Nonwoven Fabrics Handbook (edited by INDA, Association of the Nonwoven Fabrics Industry, in the U.S., Japan Nonwovens Report, 1996), which are generally used in the art.

A known method can be used for forming the nonwoven fabric layered body in the first embodiment of the invention. Examples of the method include: a method of interlacing using a needle punch, a water jet, or the like; a method of thermal fusion bonding via heat embossing, ultrasonic fusion, or the like; a method of adhesion using a variety of adhesives such as a hot melt adhesive and an urethane-based adhesive; a method of layering via resin extrusion laminating.

Of the nonwoven fabric layered bodies exemplified above, in particular, a nonwoven fabric layered body containing a melt blown nonwoven fabric and the spunbond nonwoven fabric is preferable, and a nonwoven fabric layered body in which a melt blown nonwoven fabric and the spunbond nonwoven fabric are layered via partial thermal fusion bonding (thermal compression) is more preferable.

Hereafter, a nonwoven fabric layered body of a melt blown nonwoven fabric and a spunbond nonwoven fabric, which is one preferable embodiment of the nonwoven fabric layered body in the first embodiment of the invention, is explained.

Examples of the nonwoven fabric layered body of a melt blown nonwoven fabric and a spunbond nonwoven fabric include nonwoven fabric layered bodies of: a melt blown nonwoven fabric/a spunbond nonwoven fabric; a spunbond nonwoven fabric/a melt blown nonwoven fabric/a spunbond nonwoven fabric; a spunbond nonwoven fabric/a melt blown nonwoven fabric/a melt blown nonwoven fabric /a spunbond nonwoven fabric; and a spunbond nonwoven fabric/a melt blown nonwoven fabric/a spunbond nonwoven fabric/a melt blown nonwoven fabric/a spunbond nonwoven fabric. Of these nonwoven fabric layered bodies, the configuration of a spunbond nonwoven fabric/a melt blown nonwoven fabric/a spunbond nonwoven fabric is preferable, and in a case in which the nonwoven fabric layered body with such configuration is used for medical materials, it is preferable in terms of durability or the like.

The mean fiber diameter of fibers forming a melt blown nonwoven fabric is preferably in a range of from 0.1 $\mu$m to 10 $\mu$m, more preferably in a range of from 0.5 $\mu$m to 8 $\mu$m, still more preferably in a range of from 1 $\mu$m to 5 $\mu$m, and particularly preferably in a range of from 1 $\mu$m to 4 $\mu$m. When the mean fiber diameter is within the above range, the obtained melt blown nonwoven fabric has favorable uniformity and excellent barrier performance. In addition, when the mean fiber diameter of fibers forming a melt blown nonwoven fabric is in the above range, excellent strength, barrier performance, and uniformity after electron beam irradiation are achieved, which is preferable.

The weight per unit area of a melt blown nonwoven fabric is preferably in a range of from 1 g/m$^2$ to 30 g/m$^2$, more preferably in a range of from 3 g/m$^2$ to 25 g/m$^2$, still more preferably in a range of from 5 g/m$^2$ to 20 g/m$^2$, still more preferably in a range of from 7 g/m$^2$ to 18 g/m$^2$, and particularly preferably in a range of from 10 g/m$^2$ to 17 g/m$^2$. When the weight per unit area is within the above range, excellent flexibility and barrier performance are achieved. In addition, when the weight per unit area of a melt blown nonwoven fabric is within the above range, excellent strength, barrier performance, and uniformity after electron beam irradiation are achieved, which is preferable.

Meanwhile, in a case in which a melt blown nonwoven fabric is used mainly for the purpose requiring flexibility, a heat-sealing property, and a lightweight property (e.g., a sanitary material) for which high barrier performance is not necessarily essential, the weight per unit area may be adjusted to preferably a range of from 0.5 g/m$^2$ to 5 g/m$^2$ and more preferably a range of from 0.5 g/m$^2$ to 3 g/m$^2$.

Fibers forming a melt blown nonwoven fabric are not particularly limited. However, the fibers preferably include fibers containing an ethylene-based polymer composition for the purpose of preventing reduction of strength and barrier performance after electron beam irradiation. In addition, in a case in which fibers forming a melt blown nonwoven fabric include fibers containing an ethylene-based polymer composition, it means that each of a spunbond nonwoven fabric and a melt blown nonwoven fabric contains an ethylene-based polymer. Therefore, for example, a melt blown nonwoven fabric and a spunbond nonwoven fabric can be easily joined upon joining via heat embossing treatment or the like, and the obtained nonwoven fabric layered body has excellent inter-layer adhesion strength, which is also preferable.

The ethylene-based polymer composition is preferably an ethylene-based polymer composition containing an ethylene-based polymer (a) having a melt flow rate (MFR) of from 10 g/10 minutes to 250 g/10 minutes and an ethylene-based polymer wax (b) having a weight-average molecular weight (Mw) of from 400 to 15000 at an (a):(b) mass ratio of 90:10 to 10:90.

Fibers of the above ethylene-based polymer composition containing the specific ethylene-based polymer (a) and thw specific ethylene-based polymer wax (b) each falling within the relevant range at a specific mass ratio are obtained, thereby preventing reduction of strength and barrier performance upon electron beam irradiation and achieving a thin fiber diameter and remarkably excellent spinnability.

<Ethylene-based Polymer (a)>

An ethylene-based polymer (a) that is a component of fibers forming a melt blown nonwoven fabric is a resin similar to the ethylene-based polymer (B) and it is a polymer mainly including ethylene such as an ethylene homopolymer or a copolymer of ethylene and a different $\alpha$-olefin.

The density of the ethylene-based polymer (a) is preferably in a range of from 0.870 g/cm$^3$ to 0.990 g/cm$^3$, more preferably in a range of from 0.900 g/cm$^3$ to 0.980 g/cm$^3$, still more preferably in a range of from 0.910 g/cm$^3$ to 0.980 g/cm$^3$, and most preferably in a range of from 0.940 g/cm$^3$ to 0.980 g/cm$^3$.

In general, the ethylene-based polymer (a) is a crystalline resin, which is manufactured and sold as a low-density polyethylene, a linear low-density polyethylene, a middle-density polyethylene, a high-density polyethylene, or the like.

Examples of a different $\alpha$-olefin that is copolymerized with ethylene include $\alpha$-olefins with 3 to 20 carbons such as propylene, 1-butene, 1-pentene, 1-hexene, 4-methyl-1-pentene, 1-octene, 1-decene, 1-dodecene, 1-tetradecene, 1-hexadecene, 1-octadecene, and 1-eicosene. These ethylene-based polymers may be used alone or a mixture of two or more types thereof may be used.

The melt flow rate (MFR$_{190}$ determined at a load of 2.16 kg and 190°C in accordance with ASTM D1238) of the ethylene-based polymer (a) according to the invention is not particularly limited as long as a melt blown nonwoven fabric can be manufactured using the ethylene-based polymer (a). However, it is preferably in a range of from 10 g/10 minutes to 250 g/10 minutes, more preferably in a range of from 20 g/10 minutes to 200 g/10 minutes, and still more preferably in a range of from 50 g/10 minutes to 150 g/10 minutes. The melt flow rate of the ethylene-based polymer (a) within the above range is desirable in terms of the obtained thin fiber diameter and spinnability.

As in the case of the ethylene-based polymer (B), polymers obtained by a variety of known manufacturing methods (e.g., a high-pressure method and a middle- or low-pressure method using a Ziegler catalyst or a metallocene catalyst) can be used for the ethylene-based polymer (a).

<Ethylene-based Polymer Wax (b)>

An ethylene-based polymer wax (b) that is a component of fibers forming a melt blown nonwoven fabric is a resin similar to the ethylene-based polymer wax (C) and it has a molecular weight smaller than that of the ethylene-based polymer (a). That is, the ethylene-based polymer wax (b) is a wax-type polymer.

In addition, the ethylene-based polymer wax (b) is obtained by a manufacturing method similar to that for the ethylene-based polymer wax (C).

The weight-average molecular weight (Mw) of the ethylene-based polymer wax (b) is preferably in a range of from 400 to 15,000, more preferably in a range of from 1000 to 15000, still more preferably in a range of from 6,000 to 15,000, particularly preferably in a range of from 6000 to 10000, and most preferably in a range of from 6,500 to 10,000. When the weight-average molecular weight (Mw) of the ethylene-based polymer wax (b) is within the above range, fibers of the melt blown nonwoven fabric can be easily thinned to a sufficient extent, which is preferable.

The density of the ethylene-based polymer wax (b) is preferably from 0.890 g/cm$^3$ to 0.985 g/cm$^3$, more preferably 0.910 g/cm$^3$ or more, still more preferably 0.930 g/cm$^3$ or more, still more preferably 0.950 g/cm$^3$ or more, and particularly preferably 0.960 g/cm$^3$ or more. In addition, it is preferably 0.980 g/cm$^3$ or less. In a case in which the ethylene-based polymer wax (b) having the density within the above range is used, an excellent property of being kneaded with the ethylene-based polymer (a) and excellent spinnability and temporal stability can be achieved, which is preferable. In addition, fibers are unlikely to be melted upon embossing thermal compression for layering the obtained melt blown nonwoven fabric with the spunbond nonwoven fabric, which is preferable.

<Ethylene-based Polymer Composition>

The ethylene-based polymer composition used herein is preferably an ethylene-based polymer composition containing an ethylene-based polymer composition containing an ethylene-based polymer (a) and an ethylene-based polymer wax (b) at a mass ratio of the ethylene-based polymer (a) : the ethylene-based polymer wax (b) of from 90:10 to 10:90 as described above. In addition, the (a):(b) mass ratio is more preferably from 90:10 to 50:50, still more preferably from 90:10 to 60:40, and most preferably from 90:10 to 70:30.

When the (a):(b) mass ratio is within the above range, fibers of the melt blown nonwoven fabric are sufficiently thinned, which is excellent in terms of spinnability, and fiber damage is unlikely to occur upon embossing thermal compression for layering the obtained melt blown nonwoven fabric with the spunbond nonwoven fabric.

In addition, in a different embodiment of fibers forming a melt blown nonwoven fabric, fibers containing the propylene-based polymer (c) are also preferable. In a case in which fibers forming a melt blown nonwoven fabric contain the propylene-based polymer (c), it means each of a spunbond nonwoven fabric and a melt blown nonwoven fabric contains a propylene-based polymer. Therefore, for example, a melt blown nonwoven fabric and a spunbond nonwoven fabric can be easily joined upon joining via heat embossing treatment or the like, and the obtained nonwoven fabric layered body has excellent inter-layer adhesion strength, and further, the nonwoven fabric layered body has excellent strength, which is preferable. In a case in which strength and heat resistance of the nonwoven fabric layered body are prioritized, the melt blown nonwoven fabric preferably includes fibers containing the propylene-based polymer (c).

<Propylene-based Polymer (c)>

A propylene-based polymer (c) that is a component of fibers forming a melt blown nonwoven fabric is a resin similar to the propylene-based polymer (A) and it is a polymer mainly including propylene such as a propylene homopolymer or a copolymer of propylene and a small amount of an $\alpha$-olefin. Examples of an $\alpha$-olefin that is copolymerized with propylene include an $\alpha$-olefin with 2 carbons or more (other than 3 carbons) such as pentene and preferably an $\alpha$-olefin with from 2 to 8 carbons (other than 3 carbons). Specifically examples thereof include ethylene, 1-butene, 1-pentene, 1-hexene, 1-octene, and 4-methyl-1-. One type of or two or more types of these $\alpha$-olefins may be used.

The melting point (Tm) of the propylene-based polymer (c) is not particularly limited; however, it is favorably in a range of 125°C or more, preferably in a range of 140°C or more, still more preferably in a range of 155°C or more, and particularly preferably in a range of from 157°C to 165°C.

The melt flow rate ($MFR_{230}$: ASTM D-1238; 230°C, load: 2160 g) of the propylene-based polymer (c) is not particularly limited as long as melt spinning of the propylene-based polymer (c) is possible; however, it is preferably from 10 g/10 minutes to 4000 g/10 minutes, more preferably from 50 g/10 minutes to 3000 g/10 minutes, and still more preferably from 100 g/10 minutes to 2000 g/10 minutes.

In addition, the mass ratio (Mw/Mn) of the weight-average molecular weight (Mw) and the number average molecular weight (Mn) of the propylene-based polymer (c) according to the invention is preferably form 1.5 to 5.0. More preferably, it is in a range of from 1.5 to 4.5 because favorable spinnability can be achieved and fibers having remarkably excellent fiber strength can be obtained. Mw and Mn of the propylene-based polymer (c) according to the invention are value obtained by GPC measurement, which are determined under the following conditions. Note that Mw and Mn are calculated based on the conversion method described below by creating a calibration curve using a commercially available standard monodisperse polystyrene.

Apparatus: HLC8321 GPC/HT manufactured by Tosoh Corporation
Solvent: o-dichlorobenzene
Column: TSKgel GMH6-HT × 2, TSKgel GMH6-HTLcolumn × 2(each manufactured by Tosoh Corporation)
Flow rate: 1.0 mL/minute
Sample: 0.10 mg/mL o-dichlorobenzene solution
Temperature: 140°C
Molecular weight conversion: PP conversion/universal calibration method

The coefficients for the following Mark-Houwink viscosity equation are used for calculation of universal calibration.

Polystyrene (PS) coefficient: KPS = $1.38 \times 10^{-4}$, aPS=0.70
Polypropylene (PP) coefficient: KPP = $2.42 \times 10^{-4}$, aPP=0.707

In addition, the density of the propylene-based polymer (c) in the invention is preferably in a range of from 0.895 g/cm$^3$ to 0.915 g/cm$^3$, more preferably in a range of from 0.900 g/cm$^3$ to 0.915 g/cm$^3$, and still more preferably in a range of from 0.905 g/cm$^3$ to 0.910 g/cm$^3$.

Note that the density in the invention is a value obtained by measurement in accordance with the density gradient method of JIS K7112.

In order to secure strength and barrier performance after electron beam irradiation for a resin contained in fibers constituting a melt blown nonwoven fabric, the weight-average molecular weight (Mw) after electron beam irradiation at an absorbed dose of 45 kGy is preferably 28.000 or more and particularly preferably 33,000 or more. The weight-average molecular weight (Mw) can be determined by gel permeation chromatography (GPC) in accordance with a known method.

In addition, in order to adjust the weight-average molecular weight (Mw) after electron beam irradiation to fall within the above range, for example, the methods described below can be used: (1) a method wherein the molecular weight before electron beam irradiation is set to a high level in advance for fibers of a propylene-based polymer having an increased molecular weight, thereby adjusting the weight-average molecular weight after electron beam irradiation to fall within the above range; (2) a method wherein fibers of a propylene-based polymer composition containing a stabilizer is used, thereby reduction in the molecular weight due to electron beam irradiation is prevented to adjust the weight-average molecular weight after electron beam irradiation to fall within the above range; (3) combined use of the method (1) and the method (2).

In a case in which a propylene-based polymer having an increased molecular weight is used in order to prevent a decrease in the molecular weight after electron beam irradiation, aggravation of spinnability and reduction of barrier performance due to thickening of the fiber diameter of the obtained melt blown nonwoven fabric might occur. Meanwhile, in a case in which the spinning temperature is increased in order to improve spinnability or obtain thin fibers, high temperature degradation is likely to cause the molecular weight to decrease, which might result in loss of the effects of using a propylene-based polymer having an increased molecular weight.

Further, in a case in which high temperature spinning is conducted, a stabilizer added as an additive deteriorates due to heat, which is likely to result in loss of the effects of preventing the molecular weight from decreasing upon electron beam irradiation, thereby causing reduction of strength or barrier performance.

In view of the above, in order to obtain a melt blown nonwoven fabric having a thin fiber diameter and high barrier performance while preventing its molecular weight from decreasing after electron beam irradiation, it is preferable to use a propylene-based polymer (c) containing the stabilizer described below and having a molecular weight that allows stable spinning of thin fibers at a lowered temperature. The weight-average molecular weight (Mw) of the propylene-based polymer (c) is preferably in a range of from 30,000 to 100,000, more preferably in a range of from 40,000 to 80,000, and still more preferably in a range of from 40,000 to 60,000. The weight-average molecular weight of the propylene-based polymer (c) can be adjusted to the above preferable range through degradation of a propylene-based polymer having a weight-average molecular weight exceeding the above preferable range at high temperatures upon melting in an extruder or the like. The weight-average molecular weight (Mw) can be determined by gel permeation chromatography (GPC) in accordance with a known method.

As described above, in a case in which fibers forming a melt blown nonwoven fabric contains the propylene-based polymer (c), in order to prevent reduction of strength due to reduction of the molecular weight after electron beam irradiation and to prevent reduction of barrier performance, the fibers are favorably fibers of a propylene-based polymer composition containing a stabilizer as an additive.

It is preferable to add at least one stabilizer selected from the group consisting of an organophosphorous compound, a hindered amine-based compound, and a phenolic compound. It is particularly preferable to add an organophosphorous compound. It is more preferable to add at least two types of stabilizers. It is still more preferable to add an organophosphorous compound and a hindered amine-based compound or to add an organophosphorous compound and a phenolic compound. It is particularly preferable to add, as stabilizers, an organophosphorous compound, a hindered amine-based compound, and a phenolic compound.

(A) Organophosphorous Compound

In the invention, among known compounds, an organophosphorous compound is especially used as an antioxidant stabilizer (stabilizer). Specific examples thereof include trioctyl phosphite, trilauryl phosphite, tridecylphosphite, octyl-diphenyl phosphite, tris(2,4-di-tert-butylphenyl)phosphite, triphenyl phosphite, tris(butoxyethyl)phosphite, tris(nonylphenyl)phosphite, distearylpentaerythritol diphosphite, tetra(tridecyl)-1,1,3-tris(2-methyl-5-tert-butyl-4-hydroxyphenyl)butane diphosphite, tetra(C$_{12-15}$ mixed alkyl)-4,4'-isopropylidene-diphenyl diphosphite, tetra(tridecyl)-4,4'-butylidene-bis(3-methyl-6-tert-butylphenol)diphosphite, tris(3,5-di-tert-butyl-4-hydroxyphenyl)phosphite, tris(mono/di-mixed nonylphenyl)phosphite, hydrogenated-4,4'-isopropylidene-diphenolpolyphosphite, bis(octylphenyl)·bis[4,4'-butylidene-bis(3-methyl-6-tert-butylphenol)]·1,6-hexanediol diphosphite, phenyl·4,4'-isopropylidene-diphenol·pentaerythritol diphosphite, bis(2,4-di-tert-butylphenyl)pentaerythritol diphosphite, bis(2,6-di-tert-butyl-4-methylphenyl)pentaerythritol diphosphite, tris[4,4'-isopropylidene-bis(2-tert-butylphenol)]phosphite, phenyl·diisodecylphosphite, di(nonylphenyl)pentaerythritol diphosphite), tris(1,3-di-stearoyloxyisopropyl)phosphite, 4,4'-isopropylidene-bis(2-tert-butylphenol)·di(nonylphenyl)phosphite, 9,10-di-hydro-9-oxa-10-phosphaphenanthrene-10-oxide, and tetrakis(2,4-di-tert-butylphenyl)-4,4'-biphenylenediphosphonite.

One type of the above examples may be used alone or any combination of two or more types thereof may be used. Of these, tris(2,4-di-tert-butylphenyl)phosphite, tris(mono/di-mixed nonylphenyl)phosphite, and the like are preferably used.

The amount of the above organophosphorous compound to be used is favorably from 0.001 parts by mass to 3 parts by mass and preferably from 0.001 parts by mass to 1 part by mass with respect to 100 parts by mass of the polypropylene-based polymer.

(B) Hindered Amine-based Compound

In the invention, especially a hindered amine-based compound among known compounds may be used as a light stabilizer (stabilizer). A hindered amine-based compound may be used without particular limitation; however, a hindered amine-based compound having a structure in which each of hydrogens bonded to carbons at the second and sixth positions of piperidine is substituted with a methyl can be used. Specific examples thereof include bis(2,2,6,6-tetramethyl-4-piperidine)sebacate, decanedioic acid bis(2,2,6,6-tetramethyl-4-piperidinyl), 2,4-dichloro-6-(1,1,3,3-tetramethylbutylamino)-1,3,5-triazine·N,N'-bis(2,2,6,6-tetramethyl-4-piperidyl)hexamethylenediamine polycondensate, dimethyl succinate-1-(2-hydroxyethyl)-4-hydroxy-2,2,6,6-tetramethylpiperidine polycondensate, poly[[6-(1,1,3,3-tetramethylbutyl)imino-1,3,5-triazine-2-4-diyl][(2,2,6,6-tetramethyl-4-piperi dyl)imino]hexamethylene[(2,2,6,6-tetramethyl-4-piperidyl)imino]], tetrakis(2,2,6,6-tetramethyl-4-piperidyl)-1,2,3,4-butane tetracarboxylate, 2,2,6,6-tetramethyl-4-piperidylbenzoate, bis-(1,2,2,6,6-pentamethyl-4-piperidyl)-2-(3,5-di-t-butyl-4-hydroxybenzyl)-2-n-butylmalonate, bis-(N-methyl-2,2,6,6-tetramethyl-4-piperidyl)sebacate, 1,1'-(1,2-ethanediyl)bis(3,3,5,5-tetramethylpiperazinone), (mixed 2,2,6,6-tetramethyl-4-piperidyl/tridecyl)-1,2,3,4-butane tetracarboxylate, (mixed 1,2,2,6,6-pentamethyl-4-piperidyl/tridecyl)-1,2,3,4-butane tetracarboxylate, mixed {2,2,6,6-tetramethyl-4-piperidyl/$\beta$,$\beta$,$\beta$',$\beta$'-tetramethyl-3,9-[2,4,8,10-tetraoxaspiro(5,5)undeca ne]diethyl}-1,2,3,4-butane tetracarboxylate, mixed {1,2,2,6,6-pentamethyl-4-piperidyl/$\beta$,$\beta$,$\beta$',$\beta$'-tetramethyl-3,9-[2,4,8,10-tetraoxaspiro(5,5)unde cane]diethyl}-1,2,3,4-butane tetracarboxylate, N,N'-bis(3-aminopropyl)ethylenediamine-2-4-bis[N-butyl-N-(1,2,2,6,6-pentamethyl-4-piperi dyl)amino]-6-chloro-1,3,5-triazine condensate, poly[[6-N-morpholyl-1,3,5-triazine-2-4-diyl][(2,2,6,6-tetramethyl-4-piperidyl)imino]hexamet hylene[(2,2,6,6-tetramethyl-4-piperidyl)imino]], a condensate of N,N'-bis(2,2,6,6-tetramethyl-4-piperidyl)hexamethylenediamine and 1,2-dibromoethane, and [N-(2,2,6,6-tetramethyl-4-piperidyl)-2-methyl-2-(2,2,6,6-tetramethyl-4-piperidyl)imino]propi onamide.

It is also possible to use any of these examples alone or any combination of two or more types thereof. Of these, decanedioic acid bis(2,2,6,6-tetramethyl-4-piperidinyl), 2,4-dichloro-6-(1,1,3,3-tetramethylbutylamino)-1,3,5-triazine-N,N'-bis(2,2,6,6-tetramethyl-4-piperidyl)hexamethylenediamine polycondensate, and the like are particularly preferably used.

The amount of the above hindered amine-based compound to be used is favorably from 0.001 parts by mass to 3 parts by mass and preferably from 0.001 parts by mass to 1 part by mass with respect to 100 parts by mass of the polypropylene-based polymer.

(C) Phenolic Compound

In the invention, especially a phenolic compound among known compounds may be used as an antioxidant stabilizer (stabilizer). Specific examples of phenolic antioxidants include: less hindered-type phenolic antioxidants such as 1,1,3-tris(2-methyl-4-hydroxy-5-t-butylphenyl)-butane and 4,4'-butylidene-bis(3-methyl-6-t-butylphenol); semi hindered-type phenolic antioxidants such as 3,9-bis[1,1-dimethyl-2-{$\beta$-(3-t-butyl-4-hydroxy-5-methylphenyl)propionyloxy}ethyl]-2,4,8,1 0-tetraoxaspiro[5,5]undecane; and hindered-type phenolic antioxidants such as tris(3,5-di-t-butyl-4-hydroxybenzyl)isocyanurate, n-octadecyl-3-(3',5'-di-t-butyl-4'-hydroxyphenyl)propionate, and tetrakis[methylene-3-(3',5'-di-t-butyl-4'-hydroxy-phenyl)propionate]-methane.

It is also possible to use any of these examples alone or any combination of two or more types thereof. Of these, it is preferable to use less hindered-type phenolic antioxidants such as 1,1,3-tris(2-methyl-4-hydroxy-5-t-butylphenyl)-butane having excellent effects of preventing deterioration due to electron beam irradiation.

The amount of the above phenolic compound to be used is favorably from 0.001 parts by mass to 3 parts by mass and preferably from 0.001 parts by mass to 0.5 parts by mass with respect to 100 parts by mass of a polypropylene-based polymer.

In a case in which there is concern of coloration due to electron beam irradiation, an organic or inorganic coloring pigment may be used in combination. In such case, in order to prevent negative effects such as adsorption by coloring pigment components, it is preferable to adjust the amount of the pigment to be added to fall within a range below the amount of the above stabilizer to be added.

It is preferable to use, as the above stabilizer, tris(2,4-di-tert-butylphenyl)phosphite that is an organophosphorous compound (A), decanedioic acid bis(2,2,6,6-tetramethyl-4-piperidinyl) or 2,4-dichloro-6-(1,1,3,3-tetramethylbutylamino)-1,3,5-triazine·N,N'-bis(2,2,6,6-tetramethyl-4-piperidyl)hexamethylenediamine polycondensate as a hindered amine-based compound (B), and 1,1,3-tris(2-methyl-4-hydroxy-5-t-butylphenyl)-butane as a phenolic compound (C). It is more preferable to use these compounds in combination. It is still more preferable to add at least one hindered amine-based compound selected from an organophosphorous compound of tris(2,4-di-tert-butylphenyl)phosphite, decanedioic acid bis(2,2,6,6-tetramethyl-4-piperidinyl), and 2,4-dichloro-6-(1,1,3,3-tetramethylbutylamino)-1,3,5-triazine·N,N'-bis(2,2,6,6-tetramethyl-4-piperidyl)hexamethylenediamine polycondensate, and a phenolic compound of 1,1,3-tris(2-methyl-4-hydroxy-5-t-butylphenyl)-butane.

The total amount of these stabilizers to be used is favorably from 0.01 parts by mass to 10 parts by mass and preferably from 0.03 parts by mass to 2 parts by mass with respect to 100 parts by mass of a polypropylene-based polymer. The above stabilizers might deteriorate due to high temperature degradation, and therefore, in a case in which it is necessary to compensate a loss due to deterioration, the amount of the stabilizers to be used may be increased.

In addition to the above, the following examples may be mixed as additives within the range not impairing the objectives of the invention: sulfur-based or phosphite ester-based antioxidants; benzoate-based, benzophenone-based, triazole-based, or nickel-based light stabilizers; other metal deactivators, antistatics, lubricants, mold release agents, oroganic or inorganic pigments, fillers, peroxides, foaming agents, flame retardants, nucleating agents, and gum components such as a propylene-ethylene-based copolymer gum and ethylene-butene-based copolymer gum.

The melt blown nonwoven fabric can be manufactured using the ethylene-based polymer composition or a propylene-based polymer composition containing the stabilizer by a known method of manufacturing a melt blown nonwoven fabric. Specifically, for example, the melt blown nonwoven fabric can be manufactured by a melt blowing method including conducting melt kneading of an ethylene-based polymer composition or a propylene-based polymer composition containing the stabilizer using an extruder or the like and ejecting the melt kneading product from a spinneret having a spinning nozzle while blowing away the product by a rapid and highly humid aerial flow injected from the vicinity of the spinneret, thereby allowing self-adhesive microfibers to accumulate on a collection belt to form a web. In such case, subsequent interlacing treatment may be carried out, if necessary.

Examples of a method of interlacing treatment of the accumulated web that can be used, if appropriate, include a variety of methods such as a method of heat embossing treatment using a roll, a method of ultrasonic fusion; a method of interlacing fibers using water jet, a method of fusion in a hot air through system, and a method using a needle punch.

A method of manufacturing a nonwoven fabric layered body of a melt blown nonwoven fabric and a spunbond nonwoven fabric is not particularly limited as long as a melt blown nonwoven fabric and a spunbond nonwoven fabric can be integrated to form a layered body thereby.

Specifically, for example, the following methods can be employed but the invention is not limited thereto.

(A) A method of manufacturing a two-layer layered body, including allowing fibers obtained from an ethylene-based polymer composition obtained by a melt blowing method or a propylene-based polymer composition containing a stabilizer to directly accumulate on a preliminarily obtained spunbond nonwoven fabric so as to form a melt blown nonwoven fabric and then fusing the spunbond nonwoven fabric and the melt blown nonwoven fabric via heat embossing or the like.

(B) A method of manufacturing a three-layer layered body, including allowing fibers obtained from an ethylene-based polymer composition obtained by a melt blowing method or a propylene-based polymer composition containing a stabilizer to directly accumulate on a preliminarily obtained spunbond nonwoven fabric so as to form a melt blown nonwoven fabric, further allowing fibers formed by a spunbond method to directly accumulate on the melt blown nonwoven fabric so as to form a spunbond nonwoven fabric, and then fusing the spunbond nonwoven fabric and the melt blown nonwoven fabric via heat embossing or the like.

(C) A method of manufacturing a layered body, including superimposing a preliminarily obtained spunbond nonwoven fabric and a separately manufactured melt blown nonwoven fabric and fusing both nonwoven fabrics via heating and pressurization.

(D) A method of manufacturing a layered body, including adhering a preliminarily obtained spunbond nonwoven fabric and a separately manufactured melt blown nonwoven fabric using an adhesive such as a hot melt adhesive, a solvent-based adhesive, or the like.

A method of layering a spunbond nonwoven fabric and a melt blown nonwoven fabric via thermal fusion bonding may be a method of thermal fusion bonding of the entire contact face between both nonwoven fabrics or a method of thermal fusion bonding of a part of the contact face. It is preferable to fuse a part of the contact face of both nonwoven fabric layers by a method of heat embossing. In such case, the fusion area (embossed area percentage corresponding to the area impressed by an embossing roll) is preferably from 5% to 30% and more preferably from 10% to 20% of the contact area. When the fusion area is within the above range, the nonwoven fabric layered body has an excellent balance among barrier performance, adhesion strength, and flexibility.

In a case in which heat embossing is conducted, the embossing temperature depends on the line speed or bonding pressure upon embossing; however, it is generally in a range of from 85°C to 150°C.

An example of a method other than a method of layering a spunbond nonwoven fabric and a melt blown nonwoven fabric via thermal fusion bonding is a method of layering a spunbond nonwoven fabric and a melt blown nonwoven fabric with an adhesive. Examples of a hot melt adhesive used in this method include: resin-based adhesives such as vinyl acetate-based adhesives and polyvinyl alcohol-based adhesives; and gum-based adhesives such as styrene-butadiene-based adhesives and styrene-isoprene-based adhesives. In addition, examples of a solvent-based adhesive include: gum-based adhesives such as styrene-butadiene-based adhesives, styrene-isoprene-based adhesives, and urethane-based adhesives; resin-based organic solvents or aqueous emulsion adhesives of vinyl acetate and vinyl chloride. Of these adhesives, gum-based hot melt adhesives such as styrene-isoprene-based adhesives and styrene-butadiene-based adhesives are preferable in that texture characteristic to a spunbond nonwoven fabric can be maintained.

For a nonwoven fabric layered body of a melt blown nonwoven fabric and a spunbond nonwoven fabric, inter-layer peeling strength between a melt blown nonwoven fabric layer and a spunbond nonwoven fabric layer is preferably 0.1N or more and more preferably 0.5N or more. Most preferably, no peeling occurs.

A nonwoven fabric layered body in the second embodiment of the invention contains the melt blown nonwoven fabric described below. A nonwoven fabric layered body in the second embodiment of the invention can be favorably applied to medical clothing and drapes.

A preferable embodiment of a nonwoven fabric layered body in the second embodiment of the invention is defined as the preferable embodiment of the nonwoven fabric layered body in the first embodiment of the invention described above.

<Melt Blown Nonwoven Fabric>

The melt blown nonwoven fabric of the invention includes fibers of a propylene-based polymer composition containing the propylene-based polymer (c) and at least one stabilizer selected from the group consisting of an organophosphorous compound, a hindered amine-based compound, and a phenolic compound. The stabilizer preferably includes an organophosphorous compound, more preferably includes two or more types of stabilizers, still more preferably includes an organophosphorous compound and a hindered amine-based compound or an organophosphorous compound and a phenolic compound. The stabilizer particularly preferably includes an organophosphorous compound, a hindered amine-based compound, and a phenolic compound.

In a case in which the melt blown nonwoven fabric of the invention is used as a member of, for example, a nonwoven fabric layered body, the nonwoven fabric layered body has excellent inter-layer adhesion strength. In addition, the nonwoven fabric layered body itself has excellent strength. Further, the nonwoven fabric layered body also has excellent heat resistance.

The propylene-based polymer (c) contained in fibers forming the melt blown nonwoven fabric of the invention is defined as the propylene-based polymer (c) used for a melt blown nonwoven fabric of a nonwoven fabric layered body described above. The preferable range thereof is the same as that of the propylene-based polymer (c).

The stabilizer contained in fibers forming the melt blown nonwoven fabric of the invention is defined as the stabilizer used for a melt blown nonwoven fabric of a nonwoven fabric layered body described above. The preferable range thereof is the same as that of the stabilizer described above.

EXAMPLES

The invention is described in more detail based on the Examples below. However, the invention is not limited to the Examples.

Hereafter, the invention is described more specifically with reference to the Examples and Comparative Examples of the invention. In addition, physical properties of a melt blown nonwoven fabric, a spunbond nonwoven fabric, or a nonwoven fabric layered body were determined in the manner described below in the Examples and Comparative Examples.

(1) Weight per Unit Area (g/m$^2$)

Ten pieces each having a size of 100 mm in the machine direction (MD) $\times$ 100 mm in the cross machine direction (CD) were obtained from the nonwoven fabric to calculate the mean weight per unit area.

(2) Fiber Diameter

Ten test pieces each having a size of 10 mm × 10 mm were obtained from a nonwoven fabric obtained as a spunbond nonwoven fabric. The fiber diameter was read to one decimal place by the $\mu$m using a Nikon ECLIPSE E400 microscope at a 20-fold magnification. The diameter was measured at any 20 points for each test piece to calculate the mean value.

(3) Tensile Strength (N/50 mm) and Strength INDEX

Tensile strength (N/50 mm) and Strength INDEX were determined in accordance with JIS L 1906 for a nonwoven fabric or a nonwoven fabric layered body before electron beam irradiation and a nonwoven fabric or a nonwoven fabric layered body after electron beam irradiation at an absorbed dose of 45 kGy. A test piece having 50 mm in width × 200 mm in length was obtained from a nonwoven fabric. Tensile strength was determined using a tensile tester (Autograph AGS-J manufactured by Shimadzu Corporation) at an inter-chuck distance of 100 mm and a head speed of 300 mm/min: MD: 5 points; and CD: 5 points. The mean value was calculated to determine tensile strength (N/50 mm). In addition, Strength INDEX was calculated by the following equation:

$$[\text{Equation}] \text{ Strength INDEX} = ((((\text{MD strength})^2 + (\text{CD strength})^2))/2)^{0.5}$$

(4) Air Permeability ($cm^3/cm^2/sec$)

A test piece having a size of 150 mm (MD) × 150 mm (CD) was obtained from the nonwoven fabric. Air permeability was determined using a Frazier type air permeability tester in accordance with JIS L 1096. The mean value when n=5 was designated as the measurement value.

Water Resistance (Barrier Performance)

For a nonwoven fabric layered body before electron beam irradiation and a nonwoven fabric layered body after electron beam irradiation at an absorbed dose of 45 kGy, water-resistant pressure of a nonwoven fabric layered body was determined in accordance with Method A specified in JIS L 1096 (low water pressure method). The water-resistant pressure was used as an index of water resistance (barrier performance).

(6) Evaluation of Spinnability

The number of times of thread breaking upon spinning of the spunbond nonwoven fabric in each of the Examples was determined and evaluated based on the following classification.

  A: No thread breaking
  B: From 1 to 3 times of thread breaking
  C: From 4 to 6 times of thread breaking
  D: From 7 to 9 times of thread breaking
  E: 10 times or more of thread breaking (intermittent occurrence)
  F: Constant occurrence of thread breaking

(7) Evaluation of SB Layer-MB Layer Adhesiveness

A spunbond nonwoven fabric layer (SB layer) and a melt blown nonwoven fabric layer (MB layer) were layered by the method described in the Examples. Then, a test piece 25 mm in width × 250 mm in length was obtained. The SB layer and the MB layer were peeled, and the top and bottom ends of each peeled face was gripped by a chuck (at a gripping interval of 50 mm). Strength was determined at a tensile speed of 200 mm/min. Five measurements were read at a maximum load and the mean value was designated as the measurement value. The mean value when n=3 was designated as the measurement value and evaluated based on the following classification.

  AA: Breaking of the sample at a peeling strength of 1.0 N or more, or without peeling
  A: Peeling strength of from 0.5 N to less than 1.0 N
  B: Peeling strength of from 0.1 N to less than 0.5 N

C: Peeling strength of less than 0.1 N
D: Failed measurement due to spontaneous peeling

(Example 1)

<Production of Spunbond Nonwoven Fabric (SB)>

Melt spinning was conducted at 230°C using 100 parts by mass of a propylene homopolymer (PP; MFR: 60 g/10 minutes; density: 0.910 g/cm$^3$) and 4 parts by mass of a high-density polyethylene (PE; Prime Polymer Co., Ltd.; product name: "HI-ZEX HZ2200J;" MFR: 5.2 g/10 minutes; density: 0.964 g/cm$^3$). The obtained fibers were allowed to accumulate on a collection face, followed by heat embossing. Thus, a spunbond nonwoven fabric (SB) having a fiber diameter of 18 μm and a weight per unit area of 20 g/m$^2$ was obtained.

<Production of Melt Blown Nonwoven Fabric (MB)>

Melt spinning was conducted using a mixture of 80 parts by mass of an ethylene- 1-hexene copolymer [Prime Polymer Co., Ltd.; product name: Evolue H SP50800P; density: 0.951 g/cm$^3$; MFR: 135 g/10 minutes] and 20 parts by mass of an ethylene-based polymer wax [Mitsui Chemicals, Inc.; product name: "Excelex 40800;" density: 0.980 g/cm$^3$; weight-average molecular weight: 6900; softening point: 135°C] by a melt blowing method through ejecting a melt resin through a spinneret having a nozzle with 760 holes (0.4-mmφ) at a rate of 0.15 g/minute for a single hole so that microfibers were formed and allowed to accumulate on a collection face. Thus, a melt blown nonwoven fabric (MB) having a weight per unit area of 20 g/m$^2$ was produced.

<Production of SMS Nonwoven Fabric Layered Body>

The spunbond nonwoven fabric (SB) was layered on both sides of the melt blown nonwoven fabric (MB) obtained above. The layered body was processed by thermal fusion bonding at 110°C and a linear pressure of 1 MPa via heat embossing (at an impressed area rate of 18%). Thus, an SMS nonwoven fabric layered body having a three-layer structure was obtained. Physical properties of the obtained SMS nonwoven fabric layered body were determined in the above manner. Table 1 shows the results.

(Example 2)

Nonwoven fabrics were obtained in the manner described in Example 1 except that 2 parts by mass of an ethylene-based polymer wax [Mitsui Chemicals, Inc.; product name: "HI-WAX 110P;" density: 0.922 g/cm$^3$; weight-average molecular weight: 1200; softening point: 113°C] were used for the propylene-based polymer composition, in addition to 100 parts by mass of the propylene homopolymer (PP) and 4 parts by mass of the high-density polyethylene (PE) used in Example 1. Physical properties of each obtained nonwoven fabric were determined in the above manner. Table 1 shows the results.

(Example 3)

Nonwoven fabrics were obtained in the manner described in Example 2 except that (Mitsui Chemicals, Inc.; product name: "Excelex 48070B;" density: 0.900 g/cm$^3$; weight-average molecular weight: 9000; softening point: 98°C) was used as the ethylene-based polymer wax. Physical properties of each obtained nonwoven fabric were determined in the above manner. Table 1 shows the results.

(Example 4)

Nonwoven fabrics were obtained in the manner described in Example 1 except that the mass of the high-density polyethylene (PE) was changed to 8 parts by mass. Physical properties of the obtained nonwoven fabric were determined in the above manner. Table 1 shows the results.

(Example 5)

Nonwoven fabrics were obtained in the manner described in Example 2 except that the mass of the high-density polyethylene (PE) was changed to 8 parts by mass. Physical properties of the obtained nonwoven fabric were determined in the above manner. Table 1 shows the results.

(Example 6)

Nonwoven fabrics were obtained in the manner described in Example 5 except that (Mitsui Chemicals, Inc.; product name: "Excelex 40800") was used as the ethylene-based polymer wax. Physical properties of each obtained nonwoven fabric were determined in the above manner. Table 1 shows the results.

(Example 7)

<Production of Spunbond Nonwoven Fabric (SB)>

Melt spinning was conducted at 230°C using 1 part by mass of an ethylene-based polymer wax (Mitsui Chemicals, Inc.; product name: "HI-WAX 110P;" density: 0.922 g/cm$^3$; weight-average molecular weight: 1200), in addition to 100 parts by mass of a propylene homopolymer (PP) (MFR: 60 g/10 minutes; density: 0.910 g/cm$^3$) and 4 parts by mass of a high-density polyethylene (PE; Prime Polymer Co., Ltd.; product name: "HI-ZEX HZ2200J;" MFR: 5.2 g/10 minutes; density: 0.964 g/cm$^3$). The obtained fibers were allowed to accumulate on a collection face, followed by heat embossing. Thus, a spunbond nonwoven fabric (SB) having a fiber diameter of 18 $\mu$m and a weight per unit area of 20 g/m$^2$ was obtained.

<Production of Melt Blown Nonwoven Fabric (MB)>

A thermoplastic resin composition was obtained by adding the following to 100 parts by mass of a propylene homopolymer [MFR: 1500 g/10 minutes, weight-average molecular weight (Mw): 54000]:

(A) 0.4 parts by mass of tris(2,4-di-tert-butylphenyl)phosphite [Adeka Corporation; product name: "ADK STAB 2112"] as an organophosphorous compound;
(B) 0.3 parts by mass of a 2,4-dichloro-6-(1,1,3,3-tetramethylbutylamino)-1,3,5-triazine·N,N'-bis(2,2,6,6-tetramethyl-4-piperidyl)hexamethylenediamine polycondensate [Adeka Corporation; product name: "ADK STAB LA-94G"] and 0.1 parts by mass of decanedioic acid bis(2,2,6,6-tetramethyl-4-piperidinyl) [Adeka Corporation; product name: "ADK STAB LA-77Y"] as hindered amine-based compounds; (C) 0.2 parts by mass of 1,1,3-tris(2-methyl-4-hydroxy-5-t-butylphenyl)-butane [Adeka Corporation; product name: "ADK STAB AO-30"] as a phenolic compound; and 0.3 parts by mass of 2-[5-chloro(2H)-benzotriazole-2-yl]-4-methyl-6-(tert-butyl)phenol [Adeka Corporation; product name: "ADK STAB LA-36"] as a triazole-based compound.

Melt spinning was conducted by a melt blowing method through ejecting a melt resin through a spinneret having a nozzle with 760 holes (0.4-mm$\varphi$) at a rate of 0.15 g/minute for a single hole so that microfibers were formed and allowed to accumulate on a collection face. Thus, a melt blown nonwoven fabric (MB) having a weight per unit area of 20 g/m$^2$ was produced. The weight-average molecular weight (Mw) of the melt blown nonwoven fabric (MB) after electron beam irradiation at an absorbed dose of 45 kGy was 37400.

<Production of SMS Nonwoven Fabric Layered Body>

The spunbond nonwoven fabric (SB) was layered on both sides of the melt blown nonwoven fabric (MB) obtained above. The layered body was processed by thermal fusion bonding at 120°C and a linear pressure of 1 MPa via heat embossing (at an impressed area rate of 18%). Thus, an SMS nonwoven fabric layered body having a three-layer structure was obtained. Physical properties of the obtained SMS nonwoven fabric layered body were determined in the above manner. Table 1 shows the results.

(Example 8)

Nonwoven fabrics were obtained in the manner described in Example 7 except that a melt blown nonwoven fabric (MB) was produced using only 100 parts by mass of a propylene homopolymer [MFR: 1500 g/10 minutes; weight-average molecular weight (Mw): 54000]. Physical properties of the obtained nonwoven fabric were determined in the above manner. Table 1 shows the results. The weight-average molecular weight (Mw) of the melt blown nonwoven fabric (MB) after electron beam irradiation at an absorbed dose of 45 kGy was 25,900.

(Comparative Example 1)

Nonwoven fabrics were obtained in the manner described in Example 1 except that only 100 parts by mass of a propylene homopolymer (PP) was used. Physical properties of the obtained nonwoven fabric were determined in the above manner. Table 1 shows the results.

(Comparative Example 2)

Nonwoven fabrics were obtained in the manner described in Example 3 except that the mass of the high-density polyethylene (PE) was changed to 15 parts by mass and the mass of he ethylene-based polymer wax was changed to 1 part by mass. Physical properties of the obtained nonwoven fabric were determined in the above manner. Table 1 shows the results.

(Comparative Example 3)

Nonwoven fabrics were obtained in the manner described in Example 3 except that 4 parts by mass of (Prime Polymer Co., Ltd.; product name: "ULT-ZEX 20200J;" MFR: 18.5 g/10 minutes; density: 0.918 g/cm$^3$) was used as the high-density polyethylene and the mass of the ethylene-based polymer wax was changed to 2 parts by mass. Physical properties of the obtained nonwoven fabric were determined in the above manner. Table 1 shows the results.

(Comparative Example 4)

Nonwoven fabrics were obtained in the manner described in Example 1 except that a spunbond nonwoven fabric was produced so that the fiber diameter was adjusted to 21 μm. Physical properties of the obtained nonwoven fabric were determined in the above manner. Table 1 shows the results.

Note that "SB" denotes a spunbond nonwoven fabric, "MB" denotes a melt blown nonwoven fabric, "SMS" denotes a nonwoven fabric layered body of a spunbond nonwoven fabric/a melt blown nonwoven fabric/a spunbond nonwoven fabric, "PP" denotes a propylene-based polymer, "PE" denotes an ethylene-based polymer, "wax" denotes an ethylene-based polymer wax, and "-" denotes lack of the relevant component in Table 1.

In Examples 7 and 8 in Table 1, the values "PE" columns in "MB layer" represent values of propylene-based polymers.

EP 3 214 216 B1

[Table 1]

| | | | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|---|---|
| SB layer | PP | Density (g/cm$^3$) | 0.910 | 0.910 | 0.910 | 0.910 | 0.910 | 0.910 | 0.910 | 0.910 | 0.910 | 0.910 | 0.910 | 0.910 |
| | | MFR | 60 | 60 | 60 | 60 | 60 | 60 | 60 | 60 | 60 | 60 | 60 | 60 |
| | | Content (part) | 100 | 100 | 100 | 100 | 100 | 100 | 100 | 100 | 100 | 100 | 100 | 100 |
| | PE | Density (g/cm$^3$) | 0.964 | 0.964 | 0.964 | 0.964 | 0.964 | 0.964 | 0.964 | 0.964 | - | 0.964 | 0.918 | 0.964 |
| | | MFR | 5.2 | 5.2 | 5.2 | 5.2 | 5.2 | 5.2 | 5.2 | 5.2 | - | 5.2 | 18.5 | 5.2 |
| | | Content (part) | 4 | 4 | 4 | 8 | 8 | 8 | 4 | 4 | - | 15 | 4 | 4 |
| | Wax | Density (g/cm$^3$) | - | 0.922 | 0.900 | - | 0.922 | 0.980 | 0.922 | 0.922 | - | 0.900 | 0.900 | - |
| | | Mw | - | 1200 | 9000 | - | 1200 | 6900 | 1200 | 1200 | - | 9000 | 9000 | - |
| | | Content (part) | - | 2 | 2 | - | 2 | 2 | 1 | 1 | - | 1 | 2 | - |
| | Weight per unit area (g/m$^2$) | | 20 | 20 | 20 | 20 | 20 | 20 | 20 | 20 | 20 | 20 | 20 | 20 |
| | Before irradiation | MD strength (N) | 30.1 | 29.5 | 24.1 | 27.4 | 27.7 | 23.8 | 27.2 | 27.2 | 10.8 | 12.2 | 13.7 | 31.2 |
| | | CD strength (N) | 24.9 | 22.7 | 21.0 | 22.6 | 23.3 | 19.4 | 19.5 | 19.5 | 8.8 | 8.8 | 9.4 | 22.1 |
| | | Strength INDEX(N) | 27.6 | 26.3 | 22.6 | 25.1 | 25.6 | 21.7 | 23.7 | 23.7 | 9.9 | 10.6 | 11.8 | 27.0 |
| | | Air permeability (cm$^3$/cm$^2$/sec) | 388 | 292 | 389 | 375 | 355 | 425 | 322 | 322 | 355 | 413 | 369 | 551 |
| | | Fiber diameter ($\mu$m) | 18 | 18 | 18 | 18 | 18 | 18 | 18 | 18 | 18 | 18 | 18 | 21 |

22

(continued)

| | | | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|---|---|
| | After irradiation | MD strength (N) | 23.9 | 26.1 | 20.1 | 24.2 | 25.5 | 21.9 | 25.9 | 25.9 | 9.0 | 15.4 | 12.6 | 19.5 |
| | | CD strength (N) | 19.6 | 24.7 | 16.9 | 22.3 | 22.1 | 18.3 | 18.0 | 18.0 | 9.8 | 10.4 | 10.3 | 14.6 |
| | | Strength INDEX(N) | 21.9 | 25.4 | 18.6 | 23.3 | 23.9 | 20.2 | 22.3 | 22.3 | 9.4 | 13.1 | 11.5 | 17.2 |
| | Spinnability | | C | A | A | D | B | B | A | A | A | F | E | B |
| MB layer | PE | Density (g/cm$^3$) | 0.951 | 0.951 | 0.951 | 0.951 | 0.951 | 0.951 | PP+Stabilizer | PP | 0.951 | 0.951 | 0.951 | 0.951 |
| | | MFR | 135 | 135 | 135 | 135 | 135 | 135 | 1500 | 1500 | 135 | 135 | 135 | 135 |
| | | Content (part) | 80 | 80 | 80 | 80 | 80 | 80 | 100 | 100 | 80 | 80 | 80 | 80 |
| | Wax | Density (g/cm$^3$) | 0.980 | 0.980 | 0.980 | 0.980 | 0.980 | 0.980 | - | - | 0.980 | 0.980 | 0.980 | 0.980 |
| | | Mw | 6900 | 6900 | 6900 | 6900 | 6900 | 6900 | - | - | 6900 | 6900 | 6900 | 6900 |
| | | Content (part) | 20 | 20 | 20 | 20 | 20 | 20 | - | - | 20 | 20 | 20 | 20 |
| | Weight per unit area (g/m$^2$) | | 20 | 20 | 20 | 20 | 20 | 20 | 20 | 20 | 20 | 20 | 20 | 20 |

| | | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|---|
| SMS | Weight per unit area (g/m$^2$) | 60 | 60 | 60 | 60 | 60 | 60 | 60 | 60 | 60 | 60 | 60 | 60 |
| | SB layer-MB layer adhesiveness | B | A | B | A | A | C | AA | AA | D | B | A | B |
| | Before irradiation — MD strength (N) | 78.1 | 90.0 | 67.5 | 108.5 | 125.0 | 66.6 | 146.9 | 130.6 | 20.5 | 30.5 | 48.1 | 65.4 |
| | Before irradiation — CD strength (N) | 24.2 | 27.8 | 25.7 | 26.1 | 29.9 | 23.7 | 47.9 | 51.0 | 16.7 | 22.2 | 34.0 | 42.0 |
| | Before irradiation — Strength INDEX(N) | 57.8 | 66.6 | 51.1 | 78.9 | 90.9 | 50.0 | 109.3 | 99.1 | 18.7 | 28.7 | 41.6 | 55.0 |
| | Before irradiation — Water-resistant pressure (mmH$_2$O) | 657 | 645 | 549 | 587 | 565 | 503 | 628 | 643 | Not Evaluable | 452 | 497 | 323 |
| | After irradiation — MD strength (N) | 73.2 | 81.8 | 52.1 | 92.2 | 107.5 | 64.3 | 111.5 | 78.4 | 16.2 | 33.6 | 39.4 | 56.3 |
| | After irradiation — CD strength (N) | 22.8 | 23.9 | 20.0 | 22.1 | 25.7 | 19.2 | 36.1 | 34.7 | 20.6 | 27.8 | 26.9 | 34.4 |
| | After irradiation — Strength INDEX(N) | 54.2 | 60.3 | 39.5 | 67.0 | 78.2 | 47.4 | 82.9 | 60.6 | 18.5 | 30.8 | 33.7 | 46.7 |
| | After irradiation — Water-resistant pressure (mmH$_2$O) | 620 | 665 | 552 | 567 | 548 | 511 | 597 | 385 | Not Evaluable | 448 | 492 | 362 |

EP 3 214 216 B1

As apparent from the results of Examples 1 to 6 and Comparative Examples 1 to 4 in Table 1, when the propylene-based resin composition containing no ethylene polymer was used (Comparative Example 1), Strength INDEX of the spunbond nonwoven fabric before and after electron beam irradiation was inferior to that in the Examples, and adhesiveness to the MB layer was also inferior to that in the Examples.

In addition, it is understood that even when the propylene-based resin composition contained an ethylene-based polymer, in a case in which the content of the ethylene-based polymer (B) was high (Comparative Example 2) or a low-density ethylene-based polymer was used (Comparative Example 3), spinnability of the propylene-based resin composition was inferior to that in the Examples. It is also understood that Strength INDEX of the spunbond nonwoven fabric before and after electron beam irradiation was inferior to that in the Examples.

Further, it is understood that even when the propylene-based resin composition used in the Examples was used, in a case in which air permeability of the obtained spunbond nonwoven fabric was above 500 $cm^3/cm^2/s$ (Comparative Example 4), water-resistant pressure of the layered nonwoven fabric, which had been layered on SMS, was 500 $mmH_2O$ or less, which was inferior to that in the Examples.

Therefore, it is understood that strength was sufficiently high even after electron beam irradiation, which means that aggravation of spinnability due to the presence of an ethylene-based polymer was prevented in the Examples, compared with the Comparative Examples. It is also understood that when a nonwoven fabric layered body including a melt blown nonwoven fabric was obtained, excellent inter-layer adhesion strength was achieved, and barrier performance was high.

For example, a comparison between Example 1 and Examples 2 and 5 revealed that spinnability is improved with the addition of an ethylene-based polymer wax. Further, it is understood that a nonwoven fabric layered body including a melt blown nonwoven fabric has excellent inter-layer adhesion strength.

Furthermore, a comparison between Examples 1 to 6 and Examples 7 and 8 revealed that when fibers forming a melt blown nonwoven fabric include propylene-based polymer fibers, excellent inter-layer adhesion strength is achieved, and nonwoven fabric layered body strength is also favorable.

Moreover, a comparison between Examples 7 and 8 revealed that in Example 7 in which a starting material of the melt blown nonwoven fabric is a stabilizer, water-resistant pressure was maintained at 500 $mmH_2O$ or more even after electron beam irradiation, meaning that deterioration in water-resistant pressure was prevented.

## Claims

1. Use of a spunbond nonwoven fabric as a drape or medical clothing, wherein the spunbond nonwoven fabric consists of fibers of a propylene-based polymer composition containing 100 parts by mass of a propylene-based polymer (A) and from 1 to 10 parts by mass of an ethylene-based polymer (B) having a density of from 0.93 $g/cm^3$ to 0.98 $g/cm^3$, wherein an air permeability of the spunbond nonwoven fabric, that is measured in accordance with JIS L 1096 (2010) by a Frazier type air permeability tester at a flow rate corresponding to a pressure differential of 125 Pa, is 500 $cm^3/cm^2/s$ or less.

2. The use according to claim 1, wherein the propylene-based polymer composition contains an ethylene-based polymer wax (C) having a weight-average molecular weight (Mw) of from 400 to 15,000 and a density of from 0.890 $g/cm^3$ to 0.980 $g/cm^3$.

3. The use according to claim 2, wherein a content of the ethylene-based polymer wax (C) is from 0.1 parts by mass to less than 4 parts by mass with respect to 100 parts by mass of the propylene-based polymer (A).

4. The use according to any one of claims 1 to 3, wherein a melt flow rate (MFR) of the ethylene-based polymer (B) is from 2 g/10 minutes to 25 g/10 minutes.

5. The use according to claim 1, wherein the spunbond nonwoven fabric has a weight per unit area of from 5 $g/m^2$ to 50 $g/m^2$.

6. The use according to any one of claims 1 to 5, wherein the mean fiber diameter of the fibers is 20 $\mu$m or less.

7. The use according to any one of claims 1 to 6, wherein a Strength INDEX of the spunbond nonwoven fabric after electron beam irradiation at an absorbed dose of 45 kGy is 10N or more.

8. Use of a nonwoven fabric layered body as a drape or medical clothing, wherein the nonwoven fabric layered body comprises the spunbond nonwoven fabric as defined in any one of claims 1 to 7.

9. The use according to claim 8, wherein the nonwoven fabric layered body comprises a melt blown nonwoven fabric comprising fibers of an ethylene-based polymer composition.

10. The use according to claim 9, wherein the ethylene-based polymer composition contains an ethylene-based polymer (a) having a melt flow rate (MFR) of from 10 g/10 minutes to 250 g/10 minutes and an ethylene-based polymer wax (b) having a weight-average molecular weight (Mw) of from 400 to 15000 at an (a):(b) mass ratio of from 90:10 to 10:90.

11. The use according to claim 8, wherein the nonwoven fabric layered body comprises a melt blown nonwoven fabric comprising fibers of a propylene-based polymer composition containing a propylene-based polymer (c) and at least one stabilizer selected from the group consisting of an organophosphorous compound, a hindered amine-based compound, and a phenolic compound.

12. A nonwoven fabric layered body comprising a spunbond nonwoven fabric as described in any one of claims 1 to 8 and a melt blown nonwoven fabric, wherein the melt blown nonwoven fabric comprises fibers of a propylene-based polymer composition containing a propylene-based polymer (c) and at least one stabilizer selected from the group consisting of an organophosphorous compound and a hindered amine-based compound.

13. The nonwoven fabric layered body according to claim 12, wherein the stabilizer contains an organophosphorous compound, a hindered amine-based compound, and a phenolic compound.

14. An article of medical clothing or a drape, comprising the nonwoven fabric layered body according to claim 12 or 13.


**Patentansprüche**

1. Verwendung eines Spinnvliesstoffs als Tuch oder medizinische Kleidung, wobei der Spinnvliesstoff aus Fasern einer Propylen-basierten Polymerzusammensetzung, enthaltend 100 Massenteile eines Propylen-basierten Polymers (A) und 1 bis 10 Massenteile eines Ethylen-basierten Polymers (B) mit einer Dichte von 0,93 g/cm$^3$ bis 0,98 g/cm$^3$, besteht, wobei eine Luftdurchlässigkeit des Spinnvliesstoffs, gemessen gemäß JIS L 1096 (2010) mittels eines Luftdurchlässigkeitstesters vom Frazier-Typ bei einer Strömungsrate, die einer Druckdifferenz von 125 Pa entspricht, 500 cm$^3$/cm$^2$/s oder weniger beträgt.

2. Verwendung gemäß Anspruch 1, wobei die Propylen-basierte Polymerzusammensetzung ein Ethylen-basiertes Polymerwachs (C) mit einem gewichtsmittleren Molekulargewicht (Mw) von 400 bis 15.000 und einer Dichte von 0,890 g/cm$^3$ bis 0,980 g/cm$^3$ enthält.

3. Verwendung gemäß Anspruch 2, wobei ein Gehalt des Ethylen-basierten Polymerwachs (C) 0,1 Massenteile bis weniger als 4 Massenteile, bezogen auf 100 Massenteile des Propylen-basierten Polymers (A), beträgt.

4. Verwendung gemäß einem der Ansprüche 1 bis 3, wobei eine Schmelzflussrate (MFR) des Ethylen-basierten Polymers (B) 2 g/10 Minuten bis 25 g/10 Minuten beträgt.

5. Verwendung gemäß Anspruch 1, wobei der Spinnvliesstoff ein Flächengewicht von 5 g/m$^2$ bis 50 g/m$^2$ aufweist.

6. Verwendung gemäß einem der Ansprüche 1 bis 5, wobei der mittlere Faserdurchmesser der Fasern 20 μm oder weniger beträgt.

7. Verwendung gemäß einem der Ansprüche 1 bis 6, wobei ein Festigkeitsindex des Spinnvliesstoffs nach Bestrahlung mit Elektronenstrahlen bei einer absorbierten Dosis von 45 kGy 10 N oder mehr beträgt.

8. Verwendung eines Vliesstoff-Schichtkörpers als Tuch oder medizinische Kleidung, wobei der Vliesstoff-Schichtkörper den Spinnvliesstoffs, wie in einem der Ansprüche 1 bis 7 definiert, umfasst.

9. Verwendung gemäß Anspruch 8, wobei der Vliesstoff-Schichtkörper ein schmelzgeblasenes Vlies umfasst, das Fasern einer Ethylen-basierten Polymerzusammensetzung umfasst.

10. Verwendung gemäß Anspruch 9, wobei die Ethylen-basierte Polymerzusammensetzung ein Ethylen-basiertes Polymer (a) mit einer Schmelzflussrate (MFR) von 10 g/10 Minuten bis 250 g/10 Minuten und ein Ethylen-basiertes Polymerwachs (b) mit einem gewichtsmittleren Molekulargewicht (Mw) von 400 bis 15000 bei einem Massenverhältnis (a):(b) von 90:10 bis 10:90 enthält.

**11.** Verwendung gemäß Anspruch 8, wobei der Vliesstoff-Schichtkörper ein schmelzgeblasenes Vlies umfasst, das Fasern einer Propylen-basierten Polymerzusammensetzung, enthaltend ein Propylen-basiertes Polymer (c) und mindestens einen Stabilisator, ausgewählt aus der Gruppe, bestehend aus einer Organophosphor-Verbindung, einer gehinderten Amin-basierten Verbindung und einer Phenolverbindung, umfasst.

**12.** Vliesstoff-Schichtkörper, umfassend einen Spinnvliesstoff, wie in einem der Ansprüche 1 bis 8 beschrieben, und einen schmelzgeblasenen Vliesstoff, wobei der schmelzgeblasene Vliesstoff Fasern einer Propylen-basierten Polymerzusammensetzung umfasst, die ein Propylen-basiertes Polymer (c) und mindestens einen Stabilisator, ausgewählt aus der Gruppe, bestehend aus einer Organophosphor-Verbindung und einer gehinderten Amin-basierten Verbindung, enthält.

**13.** Vliesstoff-Schichtkörper gemäß Anspruch 12, wobei der Stabilisator eine Organophosphor-Verbindung, eine gehinderte Amin-basierte Verbindung und eine Phenolverbindung enthält.

**14.** Artikel aus medizinischem Kleidungsstück oder Tuch, umfassend den Vliesstoff-Schichtkörper gemäß Anspruch 12 oder 13.


**Revendications**

**1.** Utilisation d'un tissu non-tissé filé-lié comme vêtement médical et drapé, dans laquelle le tissu non-tissé filé-lié consiste en des fibres d'une composition de polymère à base de propylène contenant 100 parties en masse d'un polymère à base de propylène (A) et de 1 à 10 parties en masse d'un polymère à base d'éthylène (B) présentant une densité allant de 0,93 g/cm$^3$ à 0,98 g/cm$^3$,
dans laquelle une perméabilité à l'air du tissu non-tissé filé-lié, qui est mesurée selon JIS L 1096 (2010) par un testeur de perméabilité à l'air de type Frazier à un débit correspondant à un différentiel de pression de 125 Pa, est de 500 cm$^3$/cm$^2$/s ou moins.

**2.** Utilisation selon la revendication 1, dans laquelle la composition de polymère à base de propylène contient une cire de polymère à base d'éthylène (C) présentant une masse moléculaire moyenne en poids (Mw) de 400 à 15 000 et une densité allant de 0,890 g/cm$^3$ à 0,980 g/cm$^3$.

**3.** Utilisation selon la revendication 2, dans laquelle une teneur de la cire de polymère à base d'éthylène (C) est de 0,1 partie en masse à moins de 4 parties en masse par rapport à 100 parties en masse du polymère à base de propylène (A).

**4.** Utilisation selon l'une quelconque des revendications 1 à 3, dans laquelle un indice de fluidité (MFR) du polymère à base d'éthylène (B) est de 2 g/10 minutes à 25 g/10 minutes.

**5.** Utilisation selon la revendication 1, dans laquelle le tissu non tissé filé-lié présente une masse par unité de surface de 5 g/m$^2$ à 50 g/m$^2$.

**6.** Utilisation selon l'une quelconque des revendications 1 à 5, dans laquelle le diamètre moyen de fibre des fibres est de 20 μm ou moins.

**7.** Utilisation selon l'une quelconque des revendications 1 à 6, dans laquelle un INDICE de Résistance du tissu non-tissé filé-lié après irradiation par faisceau électronique à une dose absorbée de 45 kGy est de 10N ou plus.

**8.** Utilisation d'un corps en couches de tissu non-tissé comme que vêtement médical et drapé, dans laquelle le corps en couches de tissu non-tissé comprend le tissu non-tissé filé-lié tel que défini selon l'une quelconque des revendications 1 à 7.

**9.** Utilisation selon la revendication 8, dans laquelle le corps en couches de tissu non tissé comprend un tissu non tissé fondu-soufflé comprenant des fibres d'une composition de polymère à base d'éthylène.

**10.** Utilisation selon la revendication 9, dans laquelle la composition de polymère à base d'éthylène contient un polymère à base d'éthylène (a) présentant un indice de fluidité (MFR) allant de 10 g/10 minutes à 250 g/10 minutes et une cire de polymère à base d'éthylène (b) présentant une masse moléculaire moyenne en poids (Mw) de 400 à 15 000 à un rapport massique (a):(b) allant de 90:10 à 10:90.

**11.** Utilisation selon la revendication 8, dans laquelle le corps en couches de tissu non tissé comprend un tissu non tissé fondu-soufflé comprenant des fibres d'une composition de polymère à base de propylène contenant un polymère à base de propylène (c) et au moins un stabilisant sélectionné dans le groupe consistant en un composé organophosphoré, un composé à base d'amine encombrée, et un composé phénolique.

**12.** Corps en couches de tissu non-tissé comprenant un tissu non-tissé filé-lié tel que décrit dans l'une quelconque des revendications 1 à 8 et un tissu non-tissé fondu-soufflé, dans lequel le tissu non-tissé fondu-soufflé comprend des fibres d'une composition de polymère à base de propylène contenant un polymère à base de propylène (c) et au moins un stabilisant sélectionné dans le groupe consistant en un composé organophosphoré et un composé à base d'amine encombrée.

**13.** Corps en couches de tissu non tissé selon la revendication 12, dans lequel le stabilisant contient un composé organophosphoré, un composé à base d'amine encombrée, et un composé phénolique.

**14.** Article de vêtement médical ou un drap, comprenant le corps en couches de tissu non tissé selon la revendication 12 ou 13.

28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2633936 B
- JP H02215848 A
- JP 2001508813 A
- JP 2010509461 A
- EP 2463428 A1
- US 2008038982 A1
- JP H10088459 A
- EP 1039007 A1
- WO 2014046070 A
- JP H08239414 A
- WO 2007114102 A
- JP S5763310 A
- JP S5883006 A
- JP H03706 A
- JP 3476793 B
- JP H04218508 A
- JP 2003105022 A
- WO 200153369 A
- WO 200127124 A
- JP H03193796 A
- JP H0241303 A

**Non-patent literature cited in the description**

- Nonwoven Fabrics Handbook. Japan Nonwovens Report. Association of the Nonwoven Fabrics Industry, 1996